# EUROPEAN PATENT SPECIFICATION

(11) **EP 0 354 184 B1**
(45) Date of publication and mention of the grant of the patent: **26.02.1997**
(21) Application number: 89810574.7
(22) Date of filing: 26.07.1989
(51) Int. Cl.: C07D 211/58, C08K 5/34, C07D 401/14

(54) **Compounds containing substituted piperidine groups for use as stabilizers for organic materials**
Substituierte Piperidinverbindungen und ihrer Verwendung als Stabilisatoren für organische Materialien
Composés de pipéridines substituées et leur utilisation comme stabilisateurs pour matériaux organiques

(30) Priority: 04.08.1988 IT 2164388
(43) Date of publication of application: 07.02.1990
(73) Proprietor: Ciba SC Holding AG, 4057 Basel (CH); CIBA-GEIGY S.p.A., 21047 Origgio (IT)
(72) Inventor: Cantatore, Giuseppe, Dr., I-70032 Bitonto (Bari) (IT); Vignali, Graziano, I-40037 Sasso Marconi (Bologna) (IT)

(56) References cited:
- EP-A- 0 124 486
- EP-A- 0 209 127
- EP-A- 0 232 224

## Description

The present invention relates to novel compounds containing substituted piperidine groups, to their use as light stabilizers, heat stabilizers and oxidation stabilizers for organic materials, particularly synthetic polymers, and to organic materials thus stabilized.

It is known that polymers undergo progressive changes in their physical properties, such as loss of mechanical strength and colour changes, when they are exposed to sunlight or other sources of ultra-violet light in the presence of oxygen. To retard the photooxidative degradation of synthetic polymers, it has been proposed to use various additives having photostabilizing properties, such as some derivatives of benzophenone and benzotriazole, nickel complexes, substituted benzoic acid esters, alkylidenemalonates, cyanoacrylates, aromatic oxamides and sterically hindered amines.

Polymeric compositions stabilized with various derivatives of diamines N,N'-disubstituted with piperidine groups have been claimed in US Patent 3,904,581 and EP-A-124 486.

In particular, the present invention relates to novel compounds of the general formula (I) 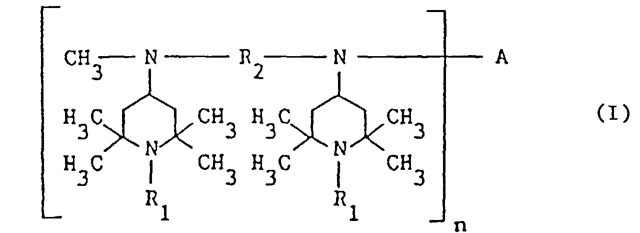 in which R₁ is hydrogen, C₁-C₈alkyl, O^{.}, OH, NO, CH₂CN, C₁-C₁₈alkoxy, C₅-C₁₂cycloalkoxy, C₃-C₆alkenyl, C₇-C₉phenylalkyl which is unsubstituted or mono-, di- or tri-substituted on the phenyl by C₁-C₄alkyl, or is C₁-C₈acyl or C₂-C₄alkyl substituted by OH in the 2-, 3- or 4-position, R₂ is C₂-C₁₂alkylene, and n is 1, 2, 3 or 4 and, if n is 1, A is hydrogen, C₂-C₁₈alkyl, C₃-C₆alkenyl, C₇-C₉phenylalkyl which is unsubstituted or mono-, di- or tri-substituted on the phenyl by C₁-C₄alkyl, or is C₂-C₄-alkyl substituted by OH in the 2-, 3- or 4-position, or A is one of the groups of the formula (IIa)- (IIb) or (IId)-(IIh) 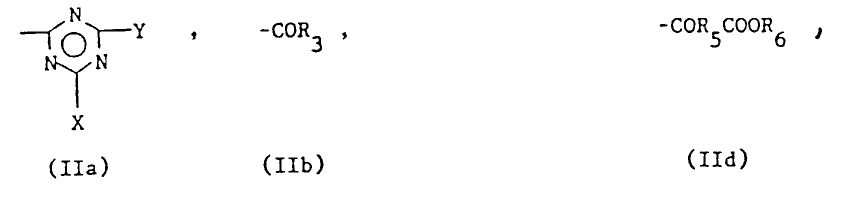 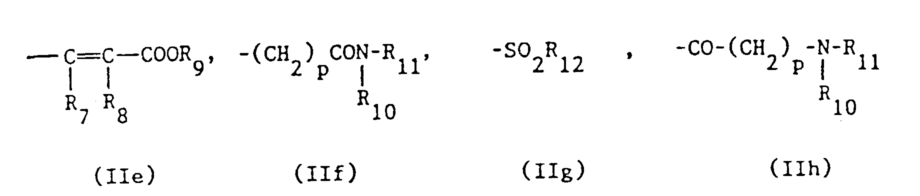 in which X and Y which can be identical or different are C₁-C₁₈alkyl, phenyl which is unsubstituted or mono-, di- or tri-substituted by C₁-C₄-alkyl, or are a group -OR₁₃, -SR₁₃ or 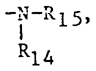 where R₁₃ is C₁-C₁₈alkyl, C₅-C₁₂cycloalkyl which is unsubstituted or mono-, di- or tri-substituted by C₁-C₄alkyl, or is C₃-C₁₈alkenyl, phenyl which is unsubstituted or mono-, di- or tri-substituted by C₁-C₄alkyl, or C₇-C₉phenylalkyl which is unsubstituted or mono-, di- or tri-substituted on the phenyl by C₁-C₄-alkyl, or is a group of the formula (III) 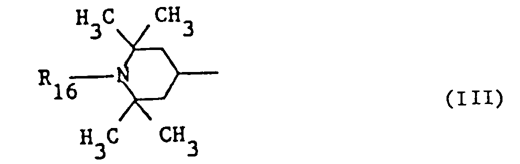 where R₁₆ corresponds to any of the definitions given for R₁, R₁₄ and R₁₅ which can be identical or different are hydrogen, C₁-C₁₈alkyl, C₅-C₁₂cycloalkyl which is unsubstituted or mono-, di- or tri-substituted by C₁-C₄alkyl, C₇-C₉phenylalkyl which is unsubstituted or mono-, di- or tri-substituted by C₁-C₄alkyl, C₂-C₄alkyl substituted in the 2-, 3- or 4-position by OH, by C₁-C₈alkoxy or by di-(C₁-C₄alkyl)-amino, or are C₃-C₁₈alkenyl, tetrahydrofurfuryl or a group of the formula (III), or R₁₄ and R₁₅, together with the nitrogen atom to which they are linked, form part of a 5-membered to 7-membered heterocyclic ring, R₃ is hydrogen, C₁-C₁₈alkyl, C₅-C₁₂cycloalkyl which is unsubstituted or mono-, di- or tri-substituted by C₁-C₄alkyl, C₂-C₁₈alkenyl, phenyl which is unsubstituted or mono-, di- or tri-substituted by C₁-C₄alkyl and/or an OH group, C₇-C₉phenylalkyl which is unsubstituted or mono-, di- or tri-substituted on the phenyl by C₁-C₄alkyl and/or an OH group, p is zero or an integer from 1 to 5, R₆ and R₉ are C₁-C₁₈alkyl, C₅-C₁₂cycloalkyl which is unsubstituted or mono-, di- or tri-substituted by C₁-C₄alkyl, C₃-C₁₈alkenyl or a group of the formula (III), R₅ is a direct bond, C₁-C₁₂alkylene, cyclohexylene or phenylene, R₇ is hydrogen, C₁-C₈alkyl or phenyl, R₈ is -CN or a group -COOR₉ with R₉ being as defined above, R₁₀ and R₁₁ which can be identical or different are as defined above for R₁₄ and R₁₅, and R₁₂ is C₁-C₁₈alkyl or phenyl which is unsubstituted or mono-, di- or tri-substituted by C₁-C₄alkyl, and, if n is 2, A is C₂-C₁₂alkylene, 2-hydroxytrimethylene, xylylene or one of the groups of the formula (IVa)-(IVe) 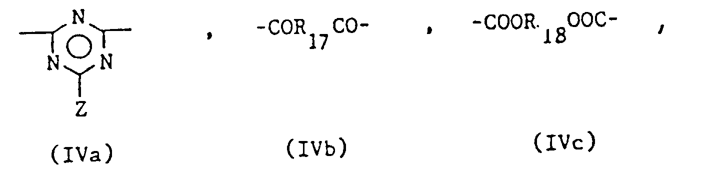 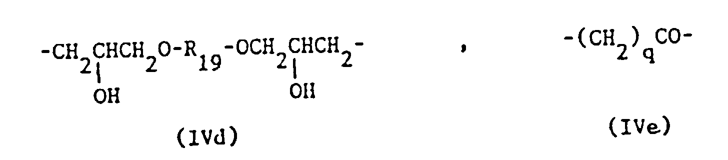 in which Z is as defined above for X and Y, R₁₇ is as defined above for R₅, R₁₈ and R₁₉ are C₂-C₁₂alkylene, C₄-C₁₂alkylene which is interrupted by 1, 2 or 3 oxygen atoms, cyclohexylene, cyclohexylenedimethylene, methylenedicyclohexylene, isopropylidenedicyclohexylene, phenylene, isopropylidenediphenylene, xylylene or a group of the formula (V) 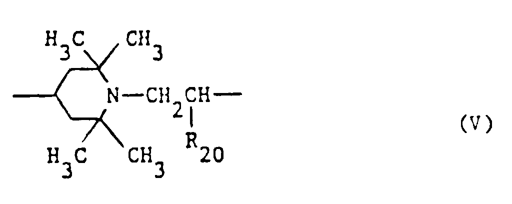 with R₂₀ being hydrogen, C₁-C₈alkyl or phenyl, and q is zero or an integer from 1 to 5, and, if n is 3, A is aliphatic C₄-C₁₈triacyl, aliphatic C₆-C₁₈triacyl substituted by one nitrogen atom, aromatic or heterocyclic triacyl having up to 18 carbon atoms or 1,3,5-triazine-2,4,6-triyl, and, if n is 4, A is aliphatic C₆-C₁₈tetraacyl, aliphatic C₁₀-C₁₈tetraacyl substituted by 2 nitrogen atoms, aromatic C₁₀-C₁₈tetraacyl or cycloaliphatic C₁₀-C₂₂tetraacyl.

Representative examples of C₁-C₈alkyl R₁, R₇, R₁₆ and R₂₀ are methyl, ethyl, propyl, butyl, isobutyl, pentyl, hexyl, heptyl and octyl. C₁-C₄alkyl, in particular methyl, is preferred.

Examples of alkyl having up to 18 carbon atoms are methyl, ethyl, propyl, isopropyl, butyl, 2-butyl, isobutyl, t-butyl, pentyl, 2-pentyl, hexyl, heptyl, octyl, 2-ethylhexyl, t-octyl, nonyl, decyl, undecyl, dodecyl, tridecyl, tetradecyl, hexadecyl and octadecyl. If A is alkyl, an alkyl group having 6 to 18 carbon atoms is preferred. Examples of OH-substituted C₂-C₄alkyl are 2-hydroxyethyl, 2-hydroxypropyl, 3-hydroxypropyl, 2-hydroxybutyl and 4-hydroxybutyl. 2-Hydroxyethyl is preferred.

Examples of C₂-C₄alkyl substituted by C₁-C₈alkoxy, preferably C₁-C₄alkoxy, in particular methoxy or ethoxy, are 2-methoxyethyl, 2-ethoxyethyl, 3-methoxypropyl, 3-ethoxypropyl, 3-butoxypropyl, 3-octoxypropyl and 4-methoxybutyl.

Examples of C₂-C₄alkyl substituted by di-(C₁-C₄alkyl)-amino, preferably dimethylamino or diethylamino, are 2-dimethylaminoethyl, 2-diethylaminoethyl, 3-dimethylaminopropyl, 3-diethylaminopropyl, 3-dibutylaminopropyl and 4-diethylaminobutyl.

Representative examples of C₁-C₁₈alkoxy R₁ and R₁₆ are methoxy, ethoxy, propoxy, isopropoxy, butoxy, isobutoxy, pentoxy, isopentoxy, hexoxy, heptoxy, octoxy, decyloxy, dodecyloxy, tetradecyloxy, hexadecyloxy and octadecyloxy. C₆-C₁₂alkoxy, in particular heptoxy or octoxy, is preferred.

Examples of unsubstituted or substituted C₅-C₁₂cycloalkyl are cyclopentyl, methylcyclopentyl, dimethylcyclopentyl, cyclohexyl, methylcyclohexyl, dimethylcyclohexyl, trimethylcyclohexyl, t-butylcyclohexyl, cyclooctyl, cyclodecyl and cyclododecyl. Cyclohexyl which is unsubstituted or substituted by C₁-C₄alkyl is preferred.

Representative examples of C₅-C₁₂cycloalkoxy R₁ and R₁₆ are cyclopentoxy, cyclohexoxy, cycloheptoxy, cyclooctoxy, cyclodecyloxy and cyclododecyloxy. Cyclopentoxy and cyclohexoxy are preferred.

Examples of alkenyl having up to 18 carbon atoms are vinyl, allyl, 2-methylallyl, hexenyl, decenyl, undecenyl and oleyl. Allyl is one of the preferred meanings of R₁ and R₁₆. If R₁, R₆, R₉-R₁₁, R₁₃-R₁₆ and A are alkenyl, the carbon atom in the 1-position is preferably a saturated carbon atom.

Examples of substituted phenyl are methylphenyl, dimethylphenyl, trimethylphenyl, t-butylphenyl, di-t-butylphenyl, 3,5-di-t-butyl-4-methylphenyl, hydroxyphenyl and 3,5-di-t-butyl-4-hydroxyphenyl.

Examples of phenylalkyl which is unsubstituted or substituted on the phenyl are benzyl, methylbenzyl, dimethylbenzyl, trimethylbenzyl, t-butylbenzyl, 2-phenylethyl and 2-(3,5-di-t-butyl-4-hydroxyphenyl)-ethyl.

Acyl R₁ and R₁₆ having up to 8 carbon atoms can be aliphatic or aromatic. Representative examples are formyl, acetyl, propionyl, butyryl, pentanoyl, hexanoyl, heptanoyl, octanoyl, benzoyl, acryloyl and crotonyl. C₁-C₈alkanoyl, C₃-C₈alkenoyl andbenzoyl are preferred. Acetyl is especially preferred.

If the groups 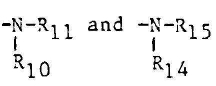 are a 5-membered to 7-membered heterocyclic group, the said heterocyclic groups preferably contain a further hetero atom, for example nitrogen or oxygen.

Representative examples are 1-pyrrolidyl, 1-piperidyl, 4-morpholinyl, 4-methyl-1-piperazinyl, 1-hexahydroazepinyl, 5,5,7-trimethyl-1-homopiperazinyl and 4,5,5,7-tetramethyl-1-homopiperazinyl.

Examples of alkylene having up to 12 carbon atoms are methylene, ethylene, propylene, trimethylene, tetramethylene, pentamethylene, 2,2-dimethyltrimethylene, hexamethylene, trimethylhexamethylene, decamethylene and dodecamethylene. R₂ is preferably ethylene or trimethylene.

Examples of C₄-C₁₂alkylene interrupted by 1, 2 or 3 oxygen atoms are 3-oxapentane-1,5-diyl, 3,6-dioxaoctane-1,8-diyl and 3,6,9-trioxaundecane-1,11-diyl.

Aliphatic C₄-C₁₈triacyl, preferably C₄-C₁₈alkanetrioyl, A can be unsubstituted or substituted by an OH group. Preferred examples are the triacyl derivatives of methanetricarboxylic acid, ethane-1,1,2-tricarboxylic acid, propane-1,2,3-tricarboxylic acid, citric acid or butane-1,2,3-tricarboxylic acid.

Aliphatic C₆-C₁₈triacyl A substituted by a nitrogen atom is, for example, a group N-[(CH₂)₁₋₅CO-]₃, preferably a group N-[(CH₂)CO-]₃.

Aromatic triacyl A having up to 18 carbon atoms is, for example, a triacyl derivative of benzene-1,2,4-tricarboxylic acid or benzene-1,3,5-tricarboxylic acid.

Heterocyclic triacyl A having up to 18 carbon atoms is, for example,

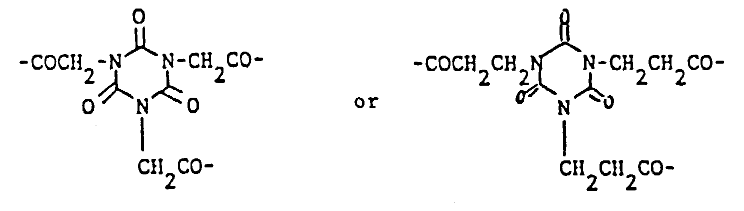

Aliphatic C₆-C₁₈tetraacyl, preferably C₆-C₈alkanetetraoyl , A is for example a tetraacyl derivative of propane-1,1,3,3-tetracarboxylic acid or butane-1,2,3,4-tetracarboxylic acid.

Aliphatic C₁₀-C₁₈tetraacyl A substituted by two nitrogen atoms is, for example, a group of the formula

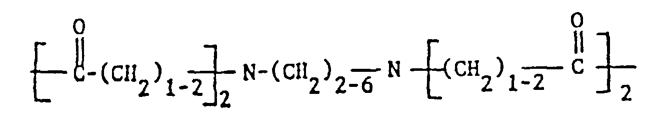

Aromatic C₁₀-C₁₈tetraacyl A is, for example, the tetraacyl derivative of benzene-1,2,4,5-tetracarboxylic acid.

Cycloaliphatic C₁₀-C₂₂tetraacyl A is, for example, one of the groups 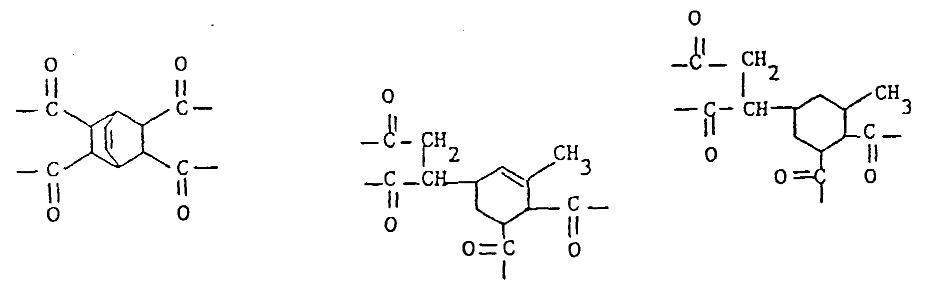 n is in particular 1, 2 or 3.
If n is 1, A is preferably a group of the formula (IIa)-(IIb) or (IId)-(IIe). If n is 2, A is preferably a group of the formula (IVa) - (IVc). If n is 3, A is preferably 1,3,5-triazine-2,4,6-triyl.

The preferred definitions of R₁ are hydrogen, C₁-C₄alkyl, -OH, C₆-C₁₂-alkoxy, C₅-C₈cycloalkoxy, allyl, benzyl, acetyl and 2-hydroxyethyl, in particular hydrogen and methyl.
Those compounds of the formula (I) are preferred in which R₂ is C₂-C₁₀-alkylene, n is 1, 2, 3 or 4 and, if n is 1, A is hydrogen, C₄-C₁₈alkyl, allyl, benzyl, 2-hydroxyethyl or 2-hydroxypropyl, or A is one of the groups of the formula (Ila)- (IIb) or (IId)-(IIh)
in which X and Y which can be identical or different are C₁-C₁₂alkyl, phenyl or a group -OR₁₃, -SR₁₃ or 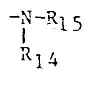 where R₁₃ is C₁-C₁₂alkyl, C₅-C₈cycloalkyl which is unsubstituted or mono-, di- or tri-substituted by C₁-C₄alkyl, C₃-C₁₂alkenyl, phenyl, benzyl or a group of the formula (III), and R₁₄ and R₁₅ which can be identical or different are hydrogen, C₁-C₁₈alkyl, C₅-C₈cycloalkyl which is unsubstituted or mono-, di- or tri-substituted by C₁-C₄alkyl, benzyl, C₂-C₃alkyl substituted in the 2-position or 3-position by OH, by C₁-C₄alkoxy or by di-(C₁-C₄alkyl)-amino, allyl, oleyl, tetrahydrofurfuryl or a group of the formula (III), or the group 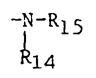 is 1-pyrrolidyl, 1-piperidyl, 4-morpholinyl or 1-hexahydroazepinyl, R₃ is hydrogen, C₁-C₁₇alkyl, C₅-C₈cycloalkyl which is unsubstituted or mono-, di- or tri-substituted by C₁-C₄alkyl, C₂-C₁₇alkenyl, phenyl which is unsubstituted or mono-, di- or tri-substituted by C₁-C₄alkyl and/or an OH group, C₇-C₈phenylalkyl which is unsubstituted or mono-, di- or tri-substituted on the phenyl by C₁-C₄alkyl and/or an OH group, p is zero or an integer from 1 to 3, R₆ and R₉ are C₁-C₁₈alkyl, C₅-C₈cycloalkyl which is unsubstituted or mono-, di- or tri-substituted by C₁-C₄alkyl, C₃-C₁₈alkenyl or a group of the formula (III), R₅ is a direct bond or C₁-C₁₀alkylene, R₇ is hydrogen or C₁-C₄alkyl, R₈ is -CN or a group -COOR₉ with R₉ being as defined above, R₁₀ and R₁₁ which can be identical or different are as defined above for R₁₄ and R₁₅, R₁₂ is C₁-C₁₂alkyl, phenyl or tolyl, and, if n is 2, A is C₂-C₁₀alkylene, 2-hydroxytrimethylene, xylylene or one of the groups of the formula (IVa)-(IVe) in which Z is as defined above for X and Y, R₁₇ is as defined above for R₅, R₁₈ and R₁₉ are C₂-C₁₀alkylene, C₄-C₁₀alkylene interrupted by 1, 2 or 3 oxygen atoms, cyclohexylenedimethylene, isopropylidenedicyclohexylene, isopropylidenediphenylene or a group of the formula (V) with R₂₀ being hydrogen or methyl, and q is zero or an integer from 1 to 3, and, if n is 3, A is aliphatic C₄-C₈triacyl, a group N(CH₂CO-)₃, a 1,3,5-triazine-2,4,6-triyl group, benzene-1,2,4-tricarbonyl, benzene-1,3,5-tricarbonyl, N,N',N"-tris(carbonylmethylene) isocyanurate or N,N',N''-tris(carbonylethylene) isocyanurate and, if n is 4, A is aliphatic C₆-C₈tetraacyl.

Those compounds of the formula (I) are particularly preferred in which R₂ is C₂-C₈alkylene, n is 1, 2 or 3 and, if n is 1, A is hydrogen, C₆-C₁₈-alkyl, benzyl or one of the groups of the formula (IIa)- (IIb) or (IId)-(IIh)
in which X and Y which can be identical or different are C₁-C₄alkyl, phenyl or a group -OR₁₃, -SR₁₃ or 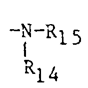 where R₁₃ is C₁-C₈alkyl, cyclohexyl which is unsubstituted or mono-, di- or tri-substituted by C₁-C₄alkyl, allyl, undecenyl, phenyl, benzyl or a group of the formula (III), R₁₄ and R₁₅ which can be identical or different are hydrogen, C₁-C₁₆alkyl, cyclohexyl which is unsubstituted or mono-, di- or tri-substituted by C₁-C₄alkyl, benzyl, C₂-C₃alkyl substituted in the 2-position or 3-position by OH, by C₁-C₄alkoxy, by dimethylamino or by diethylamino, allyl, tetrahydrofurfuryl or a group of the formula (III), or the group 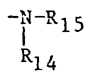 is 4-morpholinyl, R₃ is C₁-C₁₇alkyl, cyclohexyl which is unsubstituted or mono-, di- or tri-substituted by C₁-C₄alkyl, C₂-C₁₀alkenyl, phenyl, t-butylphenyl, 3,5-di-t-butyl-4-hydroxyphenyl, benzyl or 2-(3,5-di-t-butyl-4-hydroxyphenyl)ethyl, p is zero or 1, R₆ and R₉ are C₁-C₁₈alkyl, cyclohexyl which is unsubstituted or mono-, di- or tri-substituted by C₁-C₄alkyl, allyl, undecenyl, oleyl or a group of the formula (III), R₅ is a direct bond or C₁-C₈alkylene, R₇ is hydrogen or methyl, R₈ is -CN or a group -COOR₉ with R₉ being as defined above, R₁₀ and R₁₁ which can be identical or different are as defined above for R₁₄ and R₁₅, and R₁₂ is C₁-C₈alkyl, phenyl or tolyl, and, if n is 2, A is C₂-C₈alkylene, 2-hydroxytrimethylene, xylylene or one of the groups of the formula (IVa)-(IVe) in which Z is as defined above for X and Y, R₁₇ is as defined above for R₅, R₁₈ and R₁₉ are C₂-C₈alkylene, C₄-C₈alkylene interrupted by 1 or 2 oxygen atoms, cyclohexylenedimethylene, isopropylidenedicyclohexylene, isopropylidenediphenylene or a group of the formula (V) with R₂₀ being hydrogen, and q is zero or 1, and, if n is 3, A is aliphatic C₄-C₈triacyl, benzene-1,2,4-tricarbonyl, benzene-1,3,5-tricarbonyl or a 1,3,5-triazine-2,4,6-triyl group.
Those compounds of the formula (I) are of special interest in which R₂ is C₂-C₆alkylene, n is 1, 2 or 3 and, if n is 1, A is hydrogen or a group of the formula (IIa)-(IIe) or (IId)-(IIe)
in which X and Y which can be identical or different are C₁-C₈alkoxy or a group 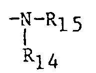 where R₁₄ and R₁₅ which can be identical or different are C₁-C₁₂alkyl, cyclohexyl, benzyl, C₂-C₃alkyl substituted in the 2-position or 3-position by methoxy, by ethoxy, by dimethylamino or by diethylamino, allyl, tetrahydrofurfuryl, 2,2,6,6-tetramethyl-4-piperidyl or 1,2,2,6,6-pentamethyl-4-piperidyl, or R₁₄ can also be hydrogen, or the group 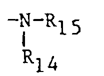 is 4-morpholinyl, R₃ is C₃-C₁₇alkyl, cyclohexyl, phenyl, 3,5-di-t-butyl-4-hydroxyphenyl or 2-(3,5-di-t-butyl-4-hydroxyphenyl)ethyl, p is zero,
R₆ and R₉ are C₂-C₁₈alkyl, cyclohexyl, trimethylcyclohexyl, t-butylcyclohexyl, 2,2,6,6-tetramethyl-4-piperidyl or 1,2,2,6,6-pentamethyl-4-piperidyl, R₃ is a direct bond or C₁-C₄alkylene, R₇ is hydrogen or methyl and R₈ is -CN or a group -COOR₉ with R₉ being as defined above, and, if n is 2, is one of the groups of the formula (IVa)-(IVd) in which Z is as defined above for X and Y, R₁₇ is a direct bond or C₁-C₈alkylene and R₁₈ and R₁₉ are C₄-C₈alkylene, cyclohexylenedimethylene, isopropylidenedicyclohexylene or isopropylidenediphenylene, and, if n is 3, A is a 1,3,5-triazine-2,4,6-triyl group.

Those compounds of the formula (I) are of particular interest in which R₁ is hydrogen or methyl, R₂ is -(CH₂ 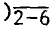 , n is 1, 2 or 3 and, if n is 1, A is hydrogen or a group of the formula (IIa)-(IIe) or (IId)-(IIe)
in which X and Y which can be identical or different are C₁-C₄alkoxy or a group 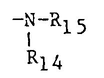 where R₁₄ and R₁₅ which can be identical or different are C₁-C₁₂alkyl, cyclohexyl, 2,2,6,6-tetramethyl-4-piperidyl or 1,2,2,6,6-pentamethyl-4-piperidyl, or R₁₄ can also be hydrogen, R₃ is C₄-C₁₇alkyl, p is zero,
R₆ and R₉ are C₂-C₁₈alkyl, cyclohexyl or t-butylcyclohexyl, R₅ is a direct bond, R₇ is hydrogen and R₈ is -CN, and, if n is 2, A is one of the groups of the formula (IVa)-(IVc) in which Z is as defined above for X and Y, R₁₇ is C₂-C₈alkylene and R₁₈ is C₄-C₆alkylene, and, if n is 3, A is a 1,3,5-triazine-2,4,6-triyl group.

The compounds of the formula (I) can be prepared by processes known per se, for example by reacting a compound of the formula (VI) 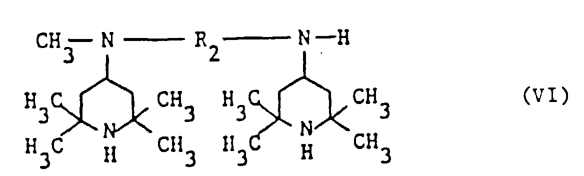 where R₂ is as defined above, with suitable alkylating or acylating agents in the appropriate molar ratios. This gives the compounds of the formula (I) where R₁ = H, from which the corresponding compounds with R₁ ≠ H can be obtained successively.

The reactions are conveniently carried out in an inert solvent, at temperatures from e.g. -20° to 200°C, preferably from -10° to 180°C.

The compounds of the formula (VI) can be prepared, for example, according to scheme 1, by reacting a compound of the formula (VII) with 2 mol of 2,2,6,6-tetramethyl-4-piperidone to give an enamine-ketimine of the formula (VIII), which is then hydrogenated in the presence of a hydrogenation catalyst such as e.g. platinum, palladium or nickel.

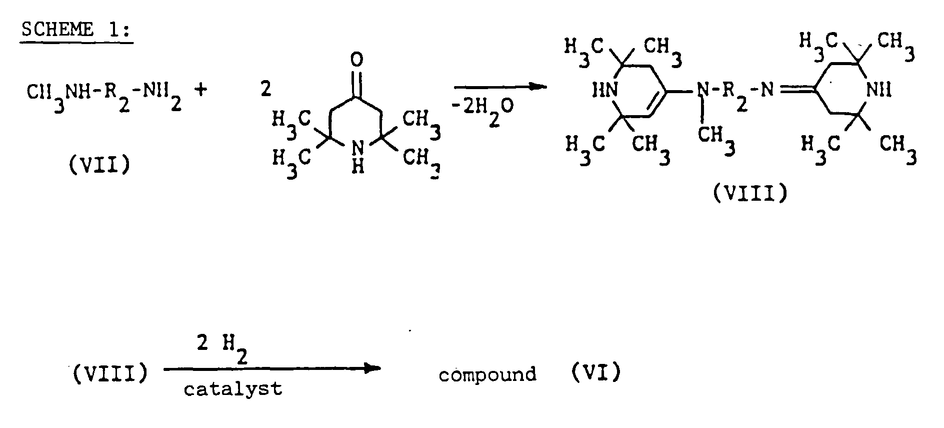

The reactions according to scheme 1 are preferably carried out in the same reactor without isolating the intermediate of the formula (VIII), operating without a solvent or in the presence of an aliphatic or aromatic hydrocarbon solvent having a boiling point from 60^{o} to 180^{o}C, preferably from 80^{o} to 140^{o}C; the hydrogenation can also be carried out e.g. in the presence of a C₁-C₄alcohol.

The compounds of the formula (VI) can, for example, also be prepared directly by catalytic hydrogenation of a mixture of 2,2,6,6-tetramethyl-4-piperidone and a compound of the formula (VII) in a molar ratio of 2/1, without a solvent or in a C₁-C₄alcohol, peferably in the presence of an organic or inorganic acid, for example benzoic acid or sulphuric acid, in a quantity from 0.001 to 0.05 mol per mol of 2,2,6,6-tetramethyl-4-piperidone.

As mentioned at the outset, the compounds of the formula (I) are highly effective in improving the light stability, heat stability and oxidation stability of organic materials, in particular synthetic polymers and copolymers.

The invention therefore also relates to a composition containing an organic material susceptible to thermal, light-induced and oxidative degradation and at least one compound of the formula (I).

Examples of such organic materials which can be stabilized are:
1. Polymers of monoolefins and diolefins, for example polypropylene, polyisobutylene, polybutene-1, polymethylpentene-1, polyisoprene or polybutadiene, as well as polymers of cycloolefins, for instance of cyclopentene or norbornene, polyethylene (which optionally can be cross-linked), for example high-density polyethylene (HDPE), low-density polyethylene (LDPE) and linear low-density polyethylene (LLDPE).
2. Mixtures of the polymers mentioned under 1), for example mixtures of polypropylene with polyisobutylene, polypropylene with polyethylene (for example PP/HDPE, PP/LDPE) and mixtures of different types of polyethylene (for example LDPE/HDPE).
3. Copolymers of monoolefins and diolefins with each other or with other vinyl monomers, such as, for example, ethylene/propylene, linear low-density polyethylene (LLDPE) and its mixtures with low-density polyethylene (LDPE), propylene/butene-1, ethylene/hexene, ethylene/ethylpentene, ethylene/heptene, ethylene/octene, propylene/isobutylene, ethylene/butene-1, propylene/butadiene, isobutylene/isoprene, ethylene/alkyl acrylates, ethylene/alkyl methacrylates, ethylene/vinyl acetate or ethylene/acrylic acid copolymers and their salts (ionomers) and terpolymers of ethylene with propylene and a diene, such as hexadiene, dicyclopentadiene or ethylidenenorbornene; as well as mixtures of such copolymers and their mixtures with polymers mentioned in 1) above, for example polypropylene/ethylenepropylene copolymers, LDPE/EVA, LDPE/EAA, LLDPE/EVA and LLDPE/EAA.
3a. Hydrocarbon resins (for example C₅-C₉) and hydrogenated modifications thereof (for example tackyfiers).
4. Polystyrene, poly-(p-methylstyrene), poly-(α-methylstyrene).
5. Copolymers of styrene or α-methylstyrene with dienes or acrylic derivatives, such as, for example, styrene/acrylonitrile, styrene/alkyl methacrylate, styrene/maleic anhydride, styrene/butadiene/ethyl acrylate, styrene/acrylonitrile/methyl acrylate; mixtures of high impact strength from styrene copolymers and another polymer, such as, for example, from a polyacrylate, a diene polymer or an ethylene/propylene/diene terpolymer; and block copolymers of styrene, such as, for example, styrene/butadiene/styrene, styrene/isoprene/styrene, styrene/ethylene/butylene/styrene or styrene/ethylene/propylene/styrene.
6. Graft copolymers of styrene or α-methylstyrene such as, for example, styrene on polybutadiene, styrene on polybutadiene-styrene or polybutadiene-acrylonitrile; styrene and acrylonitrile (or methacrylonitrile) on polybutadiene; styrene and maleic anhydride or maleimide on polybutadiene; styrene, acrylonitrile and maleic anhydride or maleimide on polybutadiene; styrene, acrylonitrile and methyl methacrylate on polybutadiene, styrene and alkyl acrylates or methacrylates on polybutadiene, styrene and acrylonitrile on ethylene/propylene/diene terpolymers, styrene and acrylonitrile on polyacrylates or polymethacrylates, styrene and acrylonitrile on acrylate/butadiene copolymers, as well as mixtures thereof with the copolymers listed under 5), for instance the copolymer mixtures known as ABS, MBS, ASA or AES polymers.
7. Halogen-containing polymers, such as polychloroprene, chlorinated rubbers, chlorinated or sulfochlorinated polyethylene, epichlorohydrin homo- and copolymers, polymers from halogen-containing vinyl compounds, as for example polyvinyl chloride, polyvinylidene chloride, polyvinyl fluoride, polyvinylidene fluoride, as well as copolymers thereof, as for example vinyl chloride/vinylidene chloride, vinyl chloride/vinyl acetate or vinylidene chloride/vinyl acetate copolymers.
8. Polymers which are derived from α,β-unsaturated acids and derivatives thereof, such as polyacrylates and polymethacrylates, polyacrylamide and polyacrylonitrile.
9. Copolymers from the monomers mentioned under 8) with each other or with other unsaturated monomers, such as, for instance, acrylonitrile/butadiene, acrylonitrile/alkyl acrylate, acrylonitrile/alkoxyalkyl acrylate or acrylonitrile/vinyl halide copolymers or acrylonitrile/alkyl methacrylate/butadiene terpolymers.
10. Polymers which are derived from unsaturated alcohols and amines, or acyl derivatives thereof or acetals thereof, such as polyvinyl alcohol, polyvinyl acetate, polyvinyl stearate, polyvinyl benzoate, polyvinyl maleate, polyvinyl butyral, polyallyl phthalate or polyallylmelamine; as well as their copolymers with olefins mentioned in 1) above.
11. Homopolymers and copolymers of cyclic ethers, such as polyalkylene glycols, polyethylene oxide, polypropylene oxide or copolymers thereof with bis-glycidyl ethers.
12. Polyacetals, such as polyoxymethylene and those polyoxymethylenes which contain ethylene oxide as a comonomer; polyacetals modified with thermoplastic polyurethanes, acrylates or MBS.
13. Polyphenylene oxides and sulfides, and mixtures of polyphenylene oxides with polystyrene or polyamides.
14. Polyurethanes which are derived from polyethers, polyesters or polybutadienes with terminal hydroxyl groups on the one hand and on the other hand aliphatic or aromatic polyisocyanates, as well as precursors thereof (polyisocyanates, polyols or prepolymers).
15. Polyamides and copolyamides which are derived from diamines and dicarboxylic acids and/or from aminocarboxylic acids or the corresponding lactams, such as polyamide 4, polyamide 6, polyamide 6/6, 6/10, 6/9, 6/12 and 4/6, polyamide 11, polyamide 12, aromatic polyamides obtained by condensation of m-xylylenediamine and adipic acid; polyamides prepared from hexamethylenediamine and isophthalic or/and terephthalic acid and optionally an elastomer as modifier, for example poly-2,4,4-trimethylhexamethylene-terephthalamide or poly-m-phenylene-isophthalamide. Further, copolymers of the aforementioned polyamides with polyolefins, olefin copolymers, ionomers or chemically bonded or grafted elastomers; or with polyethers, such as for instance, with polyethylene glycols, polypropylene glycols or polytetramethylene glycols. Polyamides or copolyamides modified with EPDM or ABS. Polyamides condensed during processing (RIM-polyamide systems).
16. Polyureas, polyimides and polyamide-imides.
17. Polyesters which are derived from dicarboxylic acids and diols and/or from hydroxycarboxylic acids or the corresponding lactones, such as polyethylene terephthalate, polybutylene terephthalate, poly-1,4-dimethylolcyclohexane terephthalate, poly-[2,2-(4-hydroxyphenyl)-propane]terephthalate and polyhydroxybenzoates as well as block copolyetheresters derived from polyethers having hydroxyl end groups.
18. Polycarbonates and polyester-carbonates.
19. Polysulfones, polyether-sulfones and polyether-ketones.
20. Crosslinked polymers which are derived from aldehydes on the one hand and phenols, ureas and melamines on the other hand, such as phenol/formaldehyde resins, urea/formaldehyde resins and melamine/formaldehyde resins.
21. Drying and non-drying alkyd resins.
22. Unsaturated polyester resins which are derived from copolyesters of saturated and unsaturated dicarboxylic acids with polyhydric alcohols and vinyl compounds as crosslinking agents, and also halogen-containing modifications thereof of low inflammability.
23. Thermosetting acrylic resins, derived from substituted acrylic esters, such as epoxy-acrylates, urethane-acrylates or polyester-acrylates.
24. Alkyd resins, polyester resins or acrylate resins in admixture with melamine resins, urea resins, polyisocyanates or epoxide resins as crosslinking agents.
25. Crosslinked epoxide resins which are derived from polyepoxides, for example from bis-glycidyl ethers or from cycloaliphatic diepoxides.
26. Natural polymers, such as cellulose, rubber, gelatine and derivatives thereof which are chemically modified in a polymer-homologous manner, such as cellulose acetates, cellulose propionates and cellulose butyrates, or cellulose ethers, such as methylcellulose; rosins and their derivatives.
27. Mixtures of polymers as mentioned above, for example PP/EPDM, polyamide 6/EPDM or ABS, PVC/EVA, PVC/ABS, PVC/MBS, PC/ASA, PC/PBT, PVC/CPE, PVC/acrylates, POM/thermoplastic PUR, PC/thermoplastic PUR, POM/acrylate, POM/MBS, PPE/HIPS, PPE/PA 6.6 and copolymers, PA/HDPE, PA/PP, PA/PPE.
28. Naturally occurring and synthetic organic materials which are pure monomeric compounds or mixtures of such compounds, for example mineral oils, animal and vegetable fats, oil and waxes, or oils, fats and waxes based on synthetic esters (e.g. phthalates, adipates, phosphates or trimellitates) and also mixtures of synthetic esters with mineral oils in any weight ratios, which materials may be used as plasticizers for polymers or as textile spinning oils, as well as aqueous emulsions of such materials.
29. Aqueous emulsions of natural or synthetic rubbers, for example natural latex or latexes of carboxylated styrene/butadiene copolymers.

The compounds of the formula (I) are particularly suitable for improving the light stability, heat stability and oxidation stability of polyolefins, especially polyethylene and polypropylene. The compounds of the formula (I) can be used in mixtures with organic materials in various proportions depending on the nature of the material to be stabilized, on the end use and on the presence of other additives.

In general, it is appropriate to use, for example, 0.01 to 5% by weight of the compounds of the formula (I), relative to the weight of the material to be stabilized, preferably from 0.05 to 1%.

The compounds of the formula (I) can be incorporated in the polymeric materials by various processes, such as e.g. dry mixing in the form of powder, or wet mixing in the form of solutions or suspensions or also in the form of a masterbatch; in such operations, the polymer can be used in the form of powder, granules, solutions, suspensions or in the form of latices.

The materials stabilized with the products of the formula (I) can be used for the production of e.g. mouldings, films, tapes, monofilaments, surface coatings and the like.

If desired, other conventional additives for synthetic polymers, such as e.g. antioxidants, UV absorbers, nickel stabilizers, pigments, fillers, plasticizers, antistatic agents, flameproofing agents, lubricants, corrosion inhibitors and metal deactivators, can be added to the mixtures of the compounds of the formula (I) with the organic materials.

Particular examples of additives which can be used in a mixture with the compounds of the formula (I) are:
1. Antioxidants
   1.1. Alkylated monophenols, for example 2,6-di-tert-butyl-4-methylphenol, 2-tert-butyl-4,6-dimethylphenol, 2,6-di-tert-butyl-4-ethylphenol, 2,6-di-tert-butyl-4-n-butylphenol, 2,6-di-tert-butyl-4-isobutylphenol, 2,6-dicyclopentyl-4-methylphenol, 2-(α-methylcyclohexyl)-4,6-dimethylphenol, 2,6-dioctadecyl-4-methylphenol, 2,4,6-tricyclohexylphenol, 2,6-di-tert-butyl-4-methoxymethylphenol, 2,6-dinonyl-4-methylphenol.
   1.2. Alkylated hydroquinones, for example 2,6-di-tert-butyl-4-methoxyphenol, 2,5-di-tert-butylhydroquinone, 2,5-di-tert-amylhydroquinone, 2,6-diphenyl-4-octadecyloxyphenol.
   1.3. Hydroxylated thiodiphenyl ethers, for example 2,2′-thiobis(6-tert-butyl-4-methylphenol), 2,2′-thiobis(4-octylphenol), 4,4′-thiobis(6-tert-butyl-3-methylphenol), 4,4′-thiobis(6-tert-butyl-2-methylphenol).
   1.4. Alkylidenebisphenols, for example 2,2′-methylenebis(6-tert-butyl-4-methylphenol), 2,2′-methylenebis(6-tert-butyl-4-ethylphenol), 2,2′-methylenebis[4-methyl-6-(α-methylcyclohexyl)phenol], 2,2′-methylenebis(4-methyl-6-cyclohexylphenol), 2,2′-methylenebis(6-nonyl-4-methylphenol), 2,2′-methylenebis(4,6-di-tert-butylphenol), 2,2′-ethylidenebis(4,6-di-tert-butylphenol), 2,2′-ethylidenebis(6-tert-butyl-4-isobutylphenol), 2,2′-methylenebis[6-(α-methylbenzyl)-4-nonylphenol], 2,2′-methylenebis[6-(α,α-dimethylbenzyl)-4-nonylphenol], 4,4′-methylenebis(2,6-di-tert-butylphenol), 4,4′-methylenebis(6-tert-butyl-2-methylphenol), 1,1-bis(5-tert-butyl-4-hydroxy-2-methylphenyl)butane, 2,6-bis(3-tert-butyl-5-methyl-2-hydroxybenzyl)-4-methylphenol, 1,1,3-tris(5-tert-butyl-4-hydroxy-2-methylphenyl)butane, 1,1-bis(5-tert-butyl-4-hydroxy-2-methylphenyl)-3-n-dodecylmercaptobutane, ethylene glycol bis[3,3-bis(3′-tert-butyl-4′-hydroxyphenyl)butyrate], bis(3-tert-butyl-4-hydroxy-5-methylphenyl)dicyclopentadiene, bis[2-(3′-tert-butyl-2′-hydroxy-5′-methylbenzyl)-6-tert-butyl-4-methylphenyl] terephthalate.
   1.5. Benzyl compounds, for example 1,3,5-tris(3,5-di-tert-butyl-4-hydroxybenzyl)-2,4,6-trimethylbenzene, bis(3,5-di-tert-butyl-4hydroxybenzyl) sulfide, isooctyl 3,5-di-tert butyl-4-hydroxybenzylmercaptoacetate, bis(4-tert-butyl-3-hydroxy-2,6-dimethylbenzyl) dithiolterephthalate, 1,3,5-tris(3,5-di-tert-butyl-4-hydroxybenzyl) isocyanurate, 1,3,5-tris(4-tert-butyl-3-hydroxy-2,6-dimethylbenzyl) isocyanurate, dioctadecyl 3,5-di-tert-butyl-4-hydroxybenzylphosphonate, calcium salt of monoethyl 3,5-di-tert-butyl-4-hydroxybenzylphosphonate, 1,3,5-tris(3,5-dicyclohexyl-4-hydroxybenzyl) isocyanurate.
   1.6. Acylaminophenols, for example lauric acid 4-hydroxyanilide, stearic acid 4-hydroxyanilide, 2,4-bis(octylmercapto)-6-(3,5-di-tert-butyl-4-hydroxyanilino)-s-triazine, octyl N-(3,5-di-tert-butyl-4-hydroxyphenyl)carbamate.
   1.7. Esters of β-(3,5-di-tert-butyl-4-hydroxyphenyl)propionic acid with mono- or polyhydric alcohols, e.g. with methanol, diethylene glycol, octadecanol, triethylene glycol, 1,6-hexanediol, pentaerythritol, neopentyl glycol, tris(hydroxyethyl) isocyanurate, thiodiethylene glycol, N,N′-bis(hydroxyethyl)oxalic acid diamide.
   1.8. Esters of β-(5-tert-butyl-4-hydroxy-3-methylphenyl)propionic acid with mono- or polyhydric alcohols, e.g. with methanol, diethylene glycol, octadecanol, triethylene glycol, 1,6-hexanediol, pentaerythritol, neopentyl glycol, tris(hydroxyethyl) isocyanurate, thiodiethylene glycol, N,N′-bis(hydroxyethyl)oxalic acid diamide.
   1.9. Esters of β-(3,5-dicyclohexyl-4-hydroxyphenyl)propionic acid with mono- or polyhydric alcohols, e.g. with methanol, diethylene glycol, octadecanol, triethylene glycol, 1,6-hexanediol, pentaerythritol, neopentyl glycol, tris(hydroxyethyl) isocyanurate, thiodiethylene glycol, N,N′-bis(hydroxyethyl)oxalic acid diamide.
   1.10 Amides of β-(3,5-di-tert-butyl-4-hydroxyphenyl)propionic acid e.g. N,N′-bis(3,5-di-tert-butyl-4-hydroxyphenylpropionyl)hexamethylenediamine, N,N′-bis(3,5-di-tert-butyl-4-hydroxyphenylpropionyl)tri-methylenediamine, N,N′-bis(3,5-di-tert-butyl-4-hydroxyphenylpropionyl)hydrazine.
2. UV absorbers and light stabilisers
   2.1. 2-(2'-Hydroxyphenyl)benzotriazoles, for example the 5'-methyl, 3',5'-di-tert-butyl, 5'-tert-butyl, 5'-(1,1,3,3-tetramethylbutyl), 5-chloro-3',5'-di-tert-butyl, 5-chloro-3'-tert-butyl-5'-methyl, 3'-sec-butyl-5'-tert-butyl, 4'-octoxy,3',5'-di-tert-amyl and 3',5'-bis(α,α-dimethylbenzyl) derivatives.
   2.2. 2-Hydroxybenzophenones, for example the 4-hydroxy, 4-methoxy, 4-octoxy, 4-decyloxy, 4-dodecyloxy, 4-benzyloxy, 4,2',4'-trihydroxy and 2'-hydroxy-4,4'-dimethoxy derivatives.
   2.3. Esters of substituted and unsubstituted benzoic acids, for example 4-tert-butylphenyl salicylate, phenyl salicylate, octylphenyl salicylate, dibenzoylresorcinol, bis(4-tert-butylbenzoyl)resorcinol, benzoylresorcinol, 2,4-di-tert-butylphenyl, 3,5-di-tert-butyl-4-hydroxybenzoate and hexadecyl 3,5-di-tert-butyl-4-hydroxybenzoate.
   2.4. Acrylates, for example ethyl α-cyano-β,β-diphenylacrylate, isooctyl α-cyano-β, β-diphenylacrylate, methyl α-carbomethoxycinnamate, methyl α-cyano-β-methyl-p-methoxycinnamate, butyl α-cyano-β-methyl-p-methoxycinnamate, methyl α-carbomethoxy-p-methoxycinnamate and N-(β-carbomethoxy-β-cyanovinyl)-2-methylindoline.
   2.5. Nickel compounds, for example nickel complexes of 2,2'-thiobis[4-(1,1,3,3-tetramethylbutyl)phenol], such as the 1:1 or 1:2 complex, with or without additional ligands such as n-butylamine, triethanolamine or N-cyclohexyldiethanolamine, nickel dibutyldithiocarbamate, nickel salts of 4-hydroxy-3,5-di-tert-butylbenzylphosphonic acid monoalkyl esters, e.g. of the methyl or ethyl ester, nickel complexes of ketoximes, e.g. of 2-hydroxy-4-methylphenyl undecyl ketoxime, nickel complexes of 1-phenyl-4-lauroyl-5-hydroxypyrazole, with or without additional ligands.
   2.6. Sterically hindered amines, for example bis(2,2,6,6-tetramethylpiperidyl) sebacate, bis(1,2,2,6,6-pentamethylpiperidyl) sebacate, bis-(1,2,2,6,6-pentamethylpiperidyl) n-butyl-3,5-di-tert-butyl-4-hydroxybenzylmalonate, the condensation product of 1-hydroxyethyl-2,2,6,6-tetramethyl-4-hydroxypiperidine and succinic acid, the condensation product of N,N′-bis(2,2,6,6-tetramethyl-4-piperidyl)hexamethylenediamine and 4-tert-octylamino-2,6-dichloro-1,3,5-triazine, tris(2,2,6,6-tetramethyl-4-piperidyl) nitrilotriacetate, tetrakis(2,2,6,6-tetramethyl-4-piperidyl) 1,2,3,4-butanetetracarboxylate, 1,1′-(1,2-ethanediyl)bis(3,3,5,5-tetramethylpiperazinone).
   2.7. Oxalic acid diamides, for example 4,4′-dioctyloxyoxanilide, 2,2′-dioctyloxy-5,5′-di-tert-butyloxanilide, 2,2′-didodecyloxy-5,5′-di-tert-butyloxanilide, 2-ethoxy-2′-ethyloxanilide, N,N′-bis(3-dimethylaminopropyl)oxalamide, 2-ethoxy-5-tert-butyl-2′-ethyloxanilide and its mixtures with 2-ethoxy-2′-ethyl-5,4′-di-tert-butyloxanilide and mixtures of ortho- and paramethoxy-disubstituted oxanilides and mixtures of o- and p- ethoxy-disubstituted oxanilides.
   2.8. 2-(2-Hydroxyphenyl)-1,3,5-triazines, for example 2,4,6-tris(2-hydroxy-4-octyloxyphenyl)-1,3,5-triazine, 2-(2-hydroxy-4-octyloxyphenyl)-4,6-bis(2,4-dimethylphenyl)-1,3,5-triazine, 2-(2,4-dihydroxyphenyl)-4,6-bis(2,4-dimethylphenyl)-1,3,5-triazine, 2,4-bis(2-hydroxy-4-propyloxyphenyl)-6-(2,4-dimethylphenyl)-1,3,5-triazine, 2-(2-hydroxy-4-octyloxyphenyl)-4,6-bis(4-methylphenyl)-1,3,5-triazine, 2-(2-hydroxy-4-dodecyloxyphenyl)-4,6-bis(2,4-dimethylphenyl)-1,3,5-triazine.
3. Metal deactivators, for example N,N′-diphenyloxalic acid diamide, N-salicylal-N′-salicyloylhydrazine, N,N′-bis(salicyloyl)hydrazine, N,N′-bis(3,5-di-tert-butyl-4-hydroxyphenylpropionyl)hydrazine, 3-salicyloylamino-1,2,4-triazole, bis(benzylidene)oxalodihydrazide.
4. Phosphites and phosphonites, for example triphenyl phosphite, diphenyl alkyl phosphites, phenyl dialkyl phosphites, tris(nonylphenyl) phosphite, trilauryl phosphite, trioctadecyl phosphite, distearyl pentaerythritol diphosphite, tris(2,4-di-tert-butylphenyl) phosphite, diisodecyl pentaerythritol diphosphite, bis(2,4-di-tert-butylphenyl) pentaerythritol diphosphite, tristearyl sorbitol triphosphite, tetrakis(2,4-di-tert-butylphenyl) 4,4′-biphenylenediphosphonite, 3,9-bis(2,4-di-tert-butylphenoxy)-2,4,8,10-tetraoxa-3,9-diphosphaspiro[5.5]undecane.
5. Peroxide scavengers, for example esters of β-thiodipropionic acid, for example the lauryl, stearyl, myristyl or tridecyl esters, mercaptobenzimidazole or the zinc salt of 2-mercaptobenzimidazole, zinc dibutyldithiocarbamate, dioctadecyl disulfide, pentaerythritol tetrakis(β-dodecylmercapto)propionate.
6. Polyamide stabilisers, for example copper salts in combination with iodides and/or phosphorus compounds and salts of divalent manganese.
7. Basic co-stabilisers, for example melamine, polyvinylpyrrolidone, dicyandiamide, triallyl cyanurate, urea derivatives, hydrazine derivatives, amines, polyamides, polyurethanes, alkali metal salts and alkaline earth metal salts of higher fatty acids, for example Ca stearate, Zn stearate, Mg stearate, Na ricinoleate and K palmitate, antimony pyrocatecholate or zinc pyrocatecholate.
8. Nucleating agents, for example 4-tert-butyl-benzoic acid, adipic acid, diphenylacetic acid.
9. Fillers and reinforcing agents, for example calcium carbonate, silicates, glass fibres, asbestos, talc, kaolin, mica, barium sulfate, metal oxides and hydroxides, carbon black, graphite.
10. Other additives, for example plasticisers, lubricants, emulsifiers, pigments, optical brighteners, flameproofing agents, antistatic agents and blowing agents.

In order to illustrate the present invention more clearly, several examples of the preparation of compounds of the formula (I) are described below; these examples are given by way of illustration only and do not imply any restriction. Particularly preferred compounds of formula (I) are those described in Examples 1, 8, 10, 13 and 18.

Example 1: Preparation of N-methyl-N,N'-bis(2,2,6,6-tetramethyl-4-piperidyl)-1,3-propanediamine.

155.2 g (1 mol) of 2,2,6,6-tetramethyl-4-piperidone, 44.1 g (0.5 mol) of N-methyl-1,3-propanediamine and 250 ml of toluene are heated under reflux with elimination of the water of reaction.

The solvent is removed in vacuo and the resulting oily residue is diluted with 250 ml of methanol and hydrogenated at 80°C in the presence of 2 g of 5% Pt on carbon under a hydrogen pressure of 50 bar until absorption is complete (about 8 hours).

After cooling to ambient temperature, the catalyst is removed by filtration and the product is separated off by distillation; boiling point 142°C/0.133 mbar.

| Analysis for C₂₂H₄₆N₄ | | | |
|---|---|---|---|
| calculated: | C = 72.07%; | H = 12.65%; | N = 15.28% |
| found: | C = 71.95%; | H = 12.59%; | N = 15.30% |

Example 2: Preparation of N-methyl-N,N'-bis(2,2,6,6-tetramethyl-4-piperidyl)-1,2-ethanediamine.

N-Methyl-N,N'-bis(2,2,6,6-tetramethyl-4-piperidyl)-1,2-ethanediamine is prepared as described in Example 1, using N-methyl-1,2-ethanediamine in place of N-methyl-1,3-propanediamine.
Boiling point 166°C/1.33 mbar.

| Analysis for C₂₁H₄₄N₄ | | | |
|---|---|---|---|
| calculated: | C = 71.53%; | H = 12.58%; | N = 15.89% |
| found: | C = 71.06%; | H = 12.52%; | N = 15.80% |

Example A: Preparation of the compound 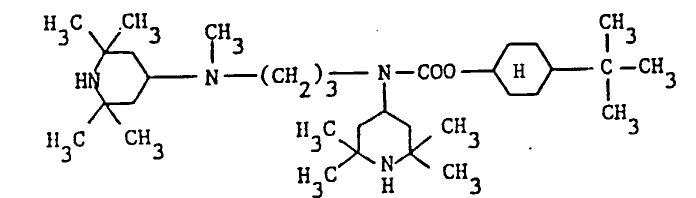 A solution of 14.4 g (0.066 mol) of 4-t-butylcyclohexyl chloroformate in 30 ml of dichloromethane is added slowly to a solution, cooled to -5°C, of 21.9 g (0.06 mol) of N-methyl-N,N'-bis(2,2,6,6-tetramethyl-4-piperidyl)-1,3-propanediamine in 100 ml of dichloromethane.

The temperature is maintained at about 0°C during the addition.

The solution is stirred for 2 hours at 10-15°C and then cooled to 0°C.

A solution of 2.8 g (0.07 mol) of sodium hydroxide in 15 ml of water is added slowly while maintaining the temperature at 0-5°C.

After one hour at this temperature, the organic phase is separated off, washed with water, dried over Na₂SO₄ and evaporated.

The residue is crystallized from acetonitrile, a product of melting point 95-97°C being obtained.

| Analysis for C₃₃H₆₄N₄O₂ | | | |
|---|---|---|---|
| calculated: | C = 72.21%; | N = 11.75%; | N = 10.21% |
| found: | C = 71.93%; | H = 11.76%; | N = 10.14% |

Examples 3-8: Following the procedure described in Example A and using the appropriate reagents, the following compounds of the formula are prepared:

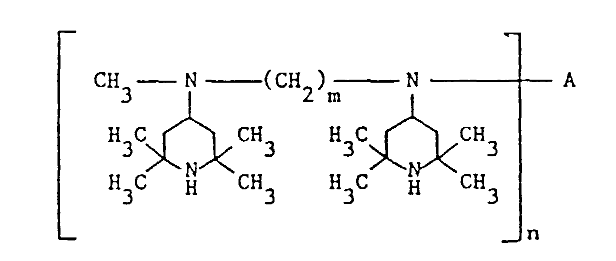

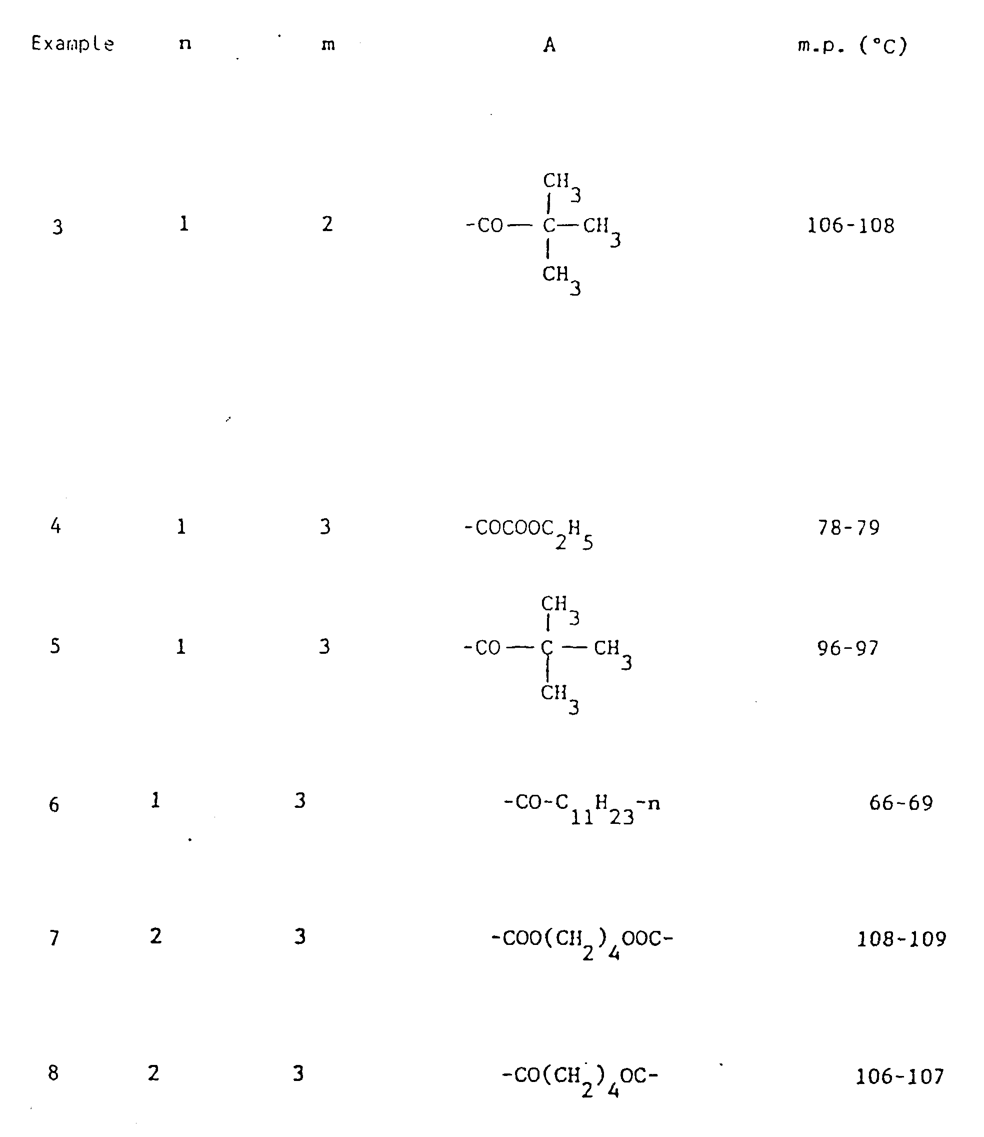

Example 9: Preparation of the compound 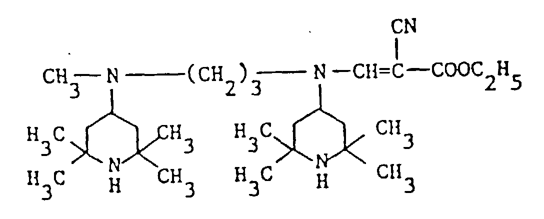 16.9 g (0.1 mol) of ethyl 2-cyano-3-ethoxyacrylate are added to a solution of 36.6 g (0.1 mol) of N-methyl-N,N'-bis(2,2,6,6-tetramethyl-4-piperidyl)-1,3-propanediamine in 80 ml of ethanol.

The solution is stirred at room temperature for 24 hours and evaporated in vacuo; the resulting residue is taken up in 100 ml of acetone, filtered and evaporated in vacuo. This gives the product as an oil.

| Analysis for C₂₈H₅₁N₅O₂ | | | |
|---|---|---|---|
| calculated: | C = 68.67%; | H = 10.50%; | N = 14.30% |
| found: | C = 68.24%; | H = 10.48%; | N = 14.20% |

Example 10: Preparation of the compound 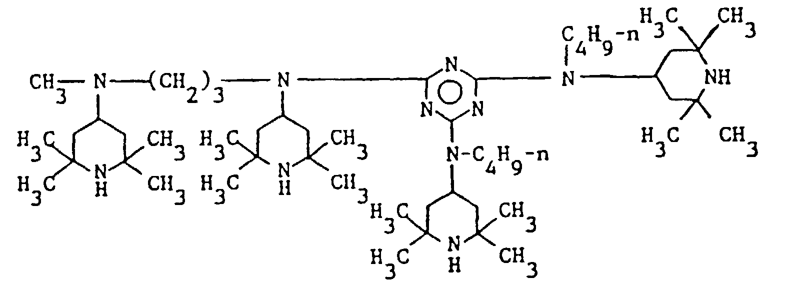 64.3 g (0.12 mol) of 2-chloro-4,6-bis[N-(2,2,6,6-tetramethyl-4-piperidyl)butylamino]-1,3,5-triazine, 43.9 g (0.12 mol) of N-methyl-N,N'-bis(2,2,6,6-tetramethyl-4-piperidyl)-1,3-propanediamine and 7.2 g (0.18 mol) of sodium hydroxide in 180 ml of mesitylene are heated under reflux for 18 hours with azeotropic elimination of the water of reaction.

The mixture is cooled to about 50°C and filtered, and the filtrate is washed with water. The solution is then dried over sodium sulphate and evaporated in vacuo (2 mbar).

The residue is taken up in n-hexane, from which the product of melting point 146-148°C crystallizes.

| Analysis for C₅₁H₉₉N₁₁ | | | |
|---|---|---|---|
| calculated: | C = 70.70%; | H = 11.52%; | N = 17.78% |
| found: | C = 70.71%; | H = 11.54%; | N = 17.76 |

Example 11: Preparation of the compound 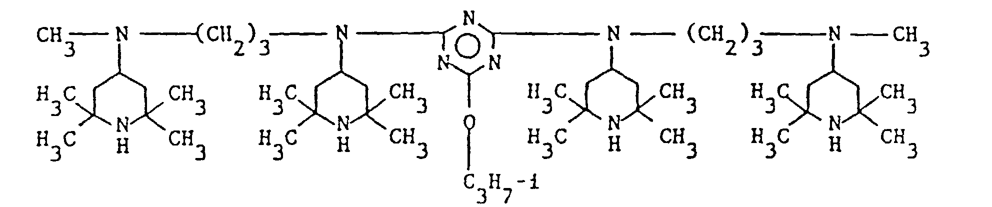 12.5 g (0.06 mol) of 2,4-dichloro-6-isopropoxy-1,3,5-triazine, 43.6 g (0.12 mol) of N-methyl-N,N'-bis(2,2,6,6-tetramethyl-4-piperidyl)-1,3-propanediamine and 100 ml of mesitylene are heated for 2 hours at 90°C. 6 g (0.15 mol) of sodium hydroxide are added, and the mixture is heated under reflux for 18 hours with elimination of the water of reaction.

The mixture is cooled to about 50°C, filtered and evaporated in vacuo (2 mbar).

The product has a melting point of 50-53°C.

| Analysis for C₅₀H₉₇N₁₁O | | | |
|---|---|---|---|
| calculated: | C = 69.16%; | H = 11.26%; | N = 17.65% |
| found: | C = 68.63%; | H = 11.21%; | N = 17.65% |

Examples 12-15: Following the procedure described in Example 11 and using the appropriate reagents, the following compounds of the formula 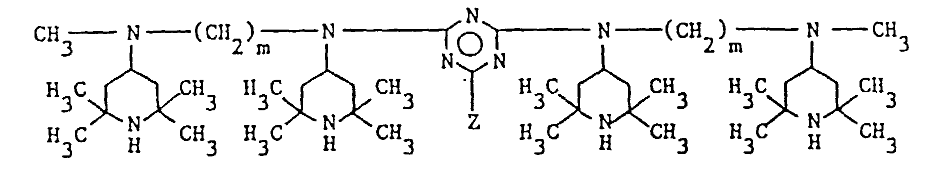 are prepared:

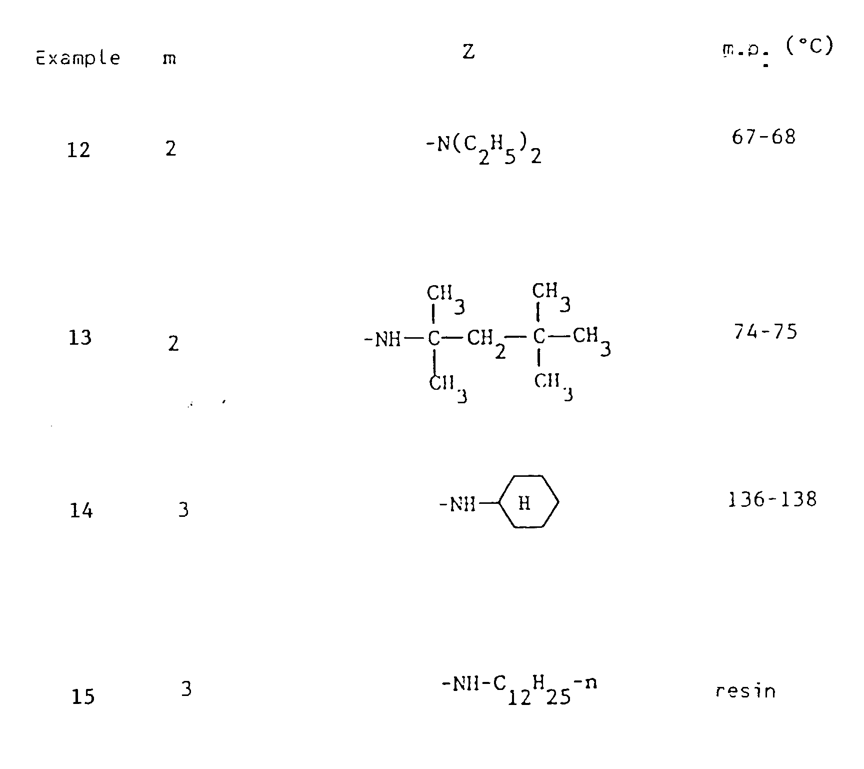

Example 16: Preparation of the compound

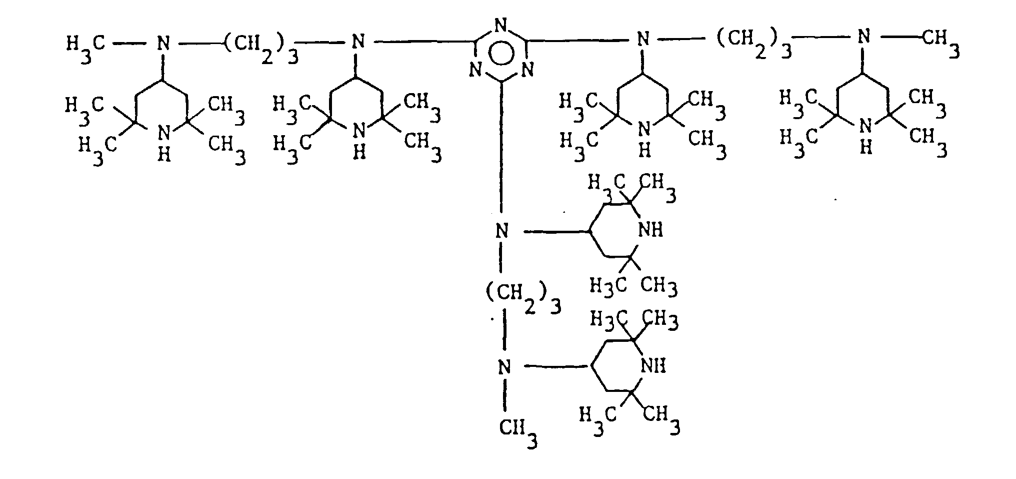

A solution of 9.2 g (0.05 mol) of cyanuric chloride in 50 ml of mesitylene is added slowly to a solution of 54.9 g (0.15 mol) of N-methyl-N,N'-bis(2,2,6,6-tetramethyl-4-piperidyl)-1,3-propanediamine in 140 ml of mesitylene.

The mixture is heated for 4 hours at 60°C, 27.6 g (0.2 mol) of potassium carbonate are added and heating is then continued under reflux for 18 hours with removal of the water of reaction.

The mixture is then cooled to about 50°C, filtered and evaporated in vacuo (2 mbar). The product has a melting point of 63-65°C.

| Analysis for C₆₉H₁₃₅N₁₅ | | | |
|---|---|---|---|
| calculated: | C = 70.54%; | H = 11.58%; | N = 17.88% |
| found: | C = 69.91%; | H = 11.50%; | N = 17.78% |

Example 17: Preparation of the compound

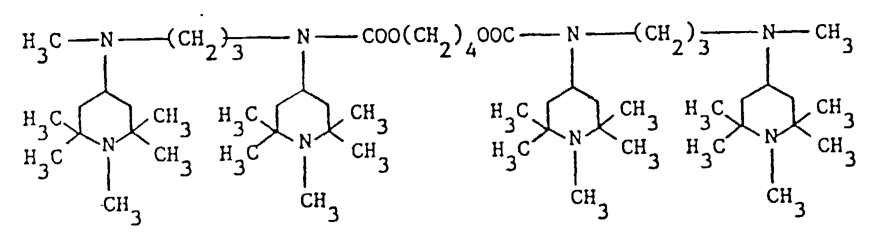

A mixture of 43.7 g (0.05 mol) of the compound from Example 9, 9 g (0.3 mol) of paraformaldehyde and 200 ml of xylene is hydrogenated at a temperature of 100°C and under a pressure of 20 bar in the presence of a suspension of 1 g of 5% Pd on carbon in 70 ml of water.

After the catalyst has been separated off by filtration, the organic phase is washed with water, dried over Na₂SO₄ and evaporated in vacuo (2 mbar).

This gives the product as a heavy oil.

| Analysis for C₅₄H₁₀₆N₈O₄ | | | |
|---|---|---|---|
| calculated: | C = 69.63%; | H = 11.47%; | N = 12.03% |
| found: | C = 69.70%; | H = 11.47%; | N = 12.02% |

Examples 18-19: Following the procedure described in Example 17 and using the appropriate reagents, the following compounds of the formula 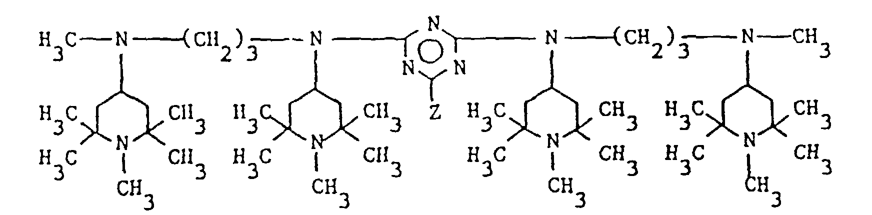 are prepared:

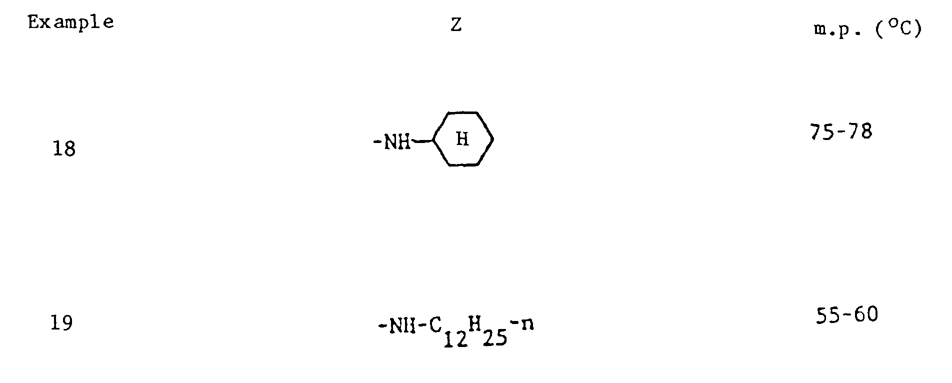

Example 20: Light-stabilizing action in polypropylene tapes. 1 g of each of the compounds indicated in Table 1, 0.5 g of tris-(2,4-di-t-butylphenyl) phosphite, 0.5 g of pentaerythritol tetrakis-[3-(3,5-di-t-butyl-4-hydroxyphenyl)propionate] and 1 g of calcium stearate are mixed in a slow mixer with 1,000 g of polypropylene powder of melt index = 2 g/10 min (measured at 230°C and 2.16 kg).

The mixtures are extruded at 200-220°C to give polymer granules which are then converted into stretched tapes of 50 µm thickness and 2.5 mm width, using a pilot-type apparatus (Leonard-Sumirago (VA) Italy) and operating under the following conditions:

| | |
|---|---|
| extruder temperature : | 210-230°C |
| head temperature : | 240-260°C |
| stretch ratio : | 1 : 6 |

The tapes thus prepared are exposed, mounted on white card, in a 65WR weather-0-meter (ASTM G26-77) at a black panel temperature of 63°C.

The residual tenacity is measured on samples, taken after various times of exposure to light, by means of a constant-speed tensometer; the exposure time (in hours) needed to halve the initial tenacity is then calculated (T₅₀).

Tapes prepared under the same conditions as indicated above, but without the addition of stabilizer, are exposed for comparison.

The results obtained are shown in Table 1.

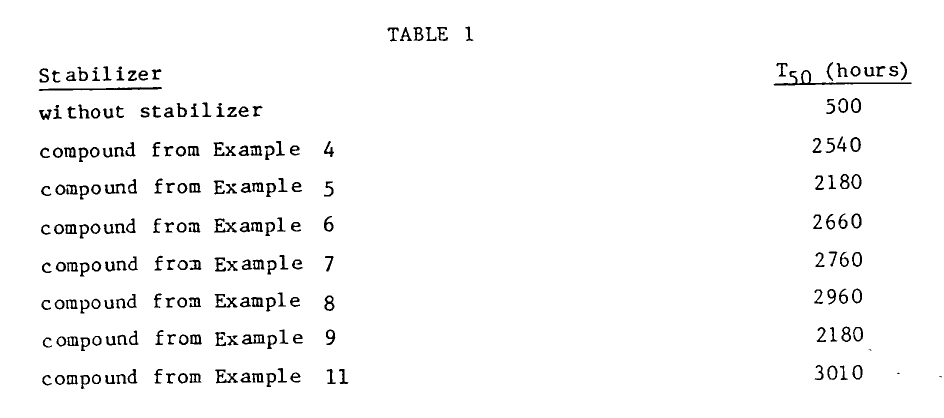

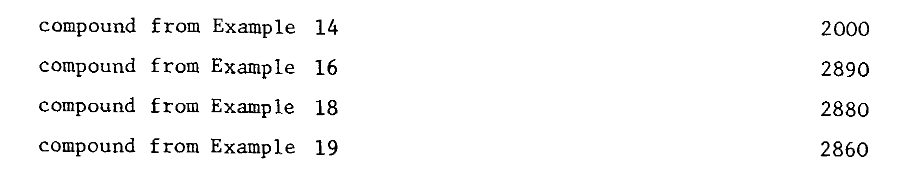

Example 21: Light-stabilizing action in polypropylene plaques. 1 g of each of the compounds indicated in Table 2, 0.5 g of tris-(2,4-di-t-butylphenyl) phosphite, 0.5 g of pentaerythritol tetrakis[3-(3,5-di-t-butyl-4-hydroxyphenyl)propionate], 1 g of phthalocyanine blue, 1 g of calcium stearate and 1,000 g of polypropylene powder of melt index = 2.1 g/10 min (measured at 230°C and 2.16 kg) are intimately mixed in a slow mixer.

The resulting mixtures are extruded at a temperature of 200-220°C to give polymer granules which are then converted into plaques of 2 mm thickness by compression-moulding at 190-220°C.

The plaques obtained are exposed in a 65WR model weather-0-meter (ASTM G 26-77) at a black panel temperature of 63°C until surface embrittlement (chalking) starts.

A polypropylene plaque prepared under the same conditions as indicated above, but without the addition of compounds of the invention, is exposed for comparison.

The exposure time (in hours) required for the onset of surface embrittlement is shown in Table 2.

**TABLE 2**

| Stabilizer | Embrittlement time (hours) |
|---|---|
| without stabilizer | 550 |
| compound from Example 4 | 3550 |
| compound from Example 5 | 3760 |
| compound from Example 6 | 4400 |
| compound from Example 7 | 4000 |
| compound from Example 8 | 3520 |
| compound from Example 9 | 3550 |

## Claims

1. A compound of the formula (I) 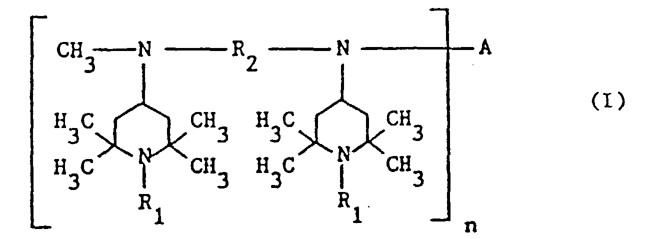 in which R₁ is hydrogen, C₁-C₈alkyl, O^{.}, OH, NO, CH₂CN, C₁-C₁₈alkoxy, C₅-C₁₂ cycloalkoxy, C₃-C₆alkenyl, C₇-C₉phenylalkyl which is unsubstituted or mono-, di- or tri-substituted on the phenyl by C₁-C₄alkyl, or is C₁-C₈acyl or C₂-C₄alkyl substituted by OH in the 2-, 3- or 4-position, R₂ is C₂-C₁₂alkylene, and n is 1, 2, 3 or 4 and, if n is 1, A is hydrogen, C₂-C₁₈alkyl, C₃-C₆alkenyl, C₇-C₉phenylalkyl which is unsubstituted or mono-; di- or tri-substituted on the phenyl by C₁-C₄alkyl, or is C₂-C₄-alkyl substituted by OH in the 2-, 3- or 4-position, or A is one of the groups of the formula (IIa)-(IIb) or (IId)-(IIh) 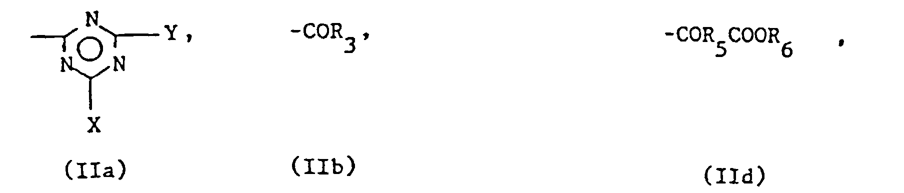 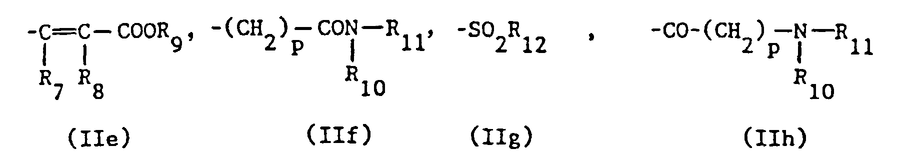 in which X and Y which can be identical or different are C₁-C₁₈alkyl, phenyl which is unsubstituted or mono-, di- or tri-substituted by C₁-C₄-alkyl, or area group -OR₁₃, -SR₁₃ or 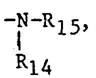 where R₁₃ is C₁-C₁₈alkyl, C₅-C₁₂cycloalkyl which is unsubstituted or mono-, di- or tri-substituted by C₁-C₄alkyl, or is C₃-C₁₈alkenyl, phenyl which is unsubstituted or mono-, di- or tri-substituted by C₁-C₄alkyl, or C₇-C₉phenylalkyl which is unsubstituted or mono-, di- or tri-substituted on the phenyl by C₁-C₄alkyl, or is a group of the formula (III) 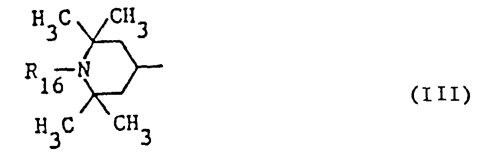 where R₁₆ corresponds to any of the definitions given for R₁, R₁₄ and R₁₅ which can be identical or different are hydrogen, C₁-C₁₈alkyl, C₅-C₁₂-cycloalkyl which is unsubstituted or mono-, di- or tri-substituted by C₁-C₄alkyl, C₇-C₉phenylalkyl which is unsubstituted or mono-, di- or tri-substituted by C₁-C₄alkyl, C₂-C₄alkyl substituted in the 2-, 3- or 4-position by OH, by C₁-C₈alkoxy or by di-(C₁-C₄alkyl)-amino, or are C₃-C₁₈-alkenyl, tetrahydrofurfuryl or a group of the formula (III), or R₁₄ and R₁₅, together with the nitrogen atom to which they are linked, form part of a 5-membered to 7-membered heterocyclic ring, R₃ is hydrogen, C₁-C₁₈-alkyl, C₅-C₁₂cycloalkyl which is unsubstituted or mono-, di- or tri-substituted by C₁-C₄alkyl, C₂-C₁₈alkenyl, phenyl which is unsubstituted or mono-, di- or tri-substituted by C₁-C₄alkyl and/or an OH group, C₇-C₉-phenylalkyl which is unsubstituted or mono-, di- or tri-substituted on the phenyl by C₁-C₄alkyl and/or an OH group, p is zero or an integer from 1 to 5, R₆ and R₉ are C₁-C₁₈alkyl, C₅-C₁₂cycloalkyl which is unsubstituted or mono-, di- or tri-substituted by C₁-C₄alkyl, C₃-C₁₈-alkenyl or a group of the formula (III), R₅ is a direct bond, C₁-C₁₂-alkylene, cyclohexylene or phenylene, R₇ is hydrogen, C₁-C₈alkyl or phenyl, R₈ is -CN or a group -COOR₉ with R₉ being as defined above, R₁₀ and R₁₁ which can be identical or different are as defined above for R₁₄ and R₁₅, and R₁₂ is C₁-C₁₈alkyl or phenyl which is unsubstituted or mono-, di- or tri-substituted by C₁-C₄alkyl, and, if n is 2, A is C₂-C₁₂alkylene, 2-hydroxytrimethylene, xylylene or one of the groups of the formula (IVa)-(IVe) 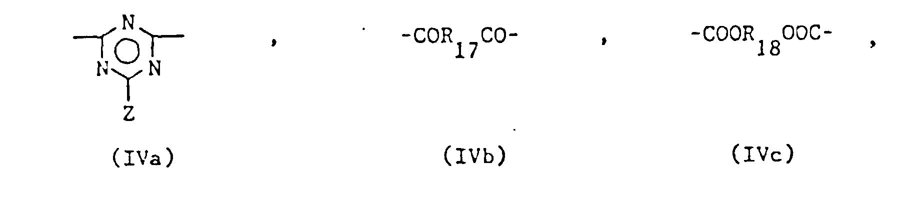 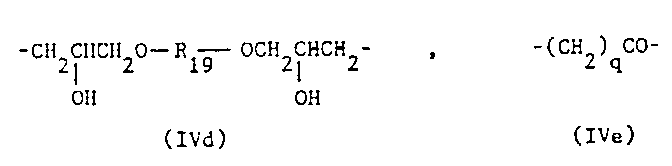 in which Z is as defined above for X and Y, R₁₇ is as defined above for R₅, R₁₈ and R₁₉ are C₂-C₁₂alkylene, C₄-C₁₂alkylene which is interrupted by 1, 2 or 3 oxygen atoms, cyclohexylene, cyclohexylenedimethylene, methylenedicyclohexylene, isopropylideneicyclohexylene, phenylene, isopropylidenediphenylene, xylylene or a group of the formula (V) 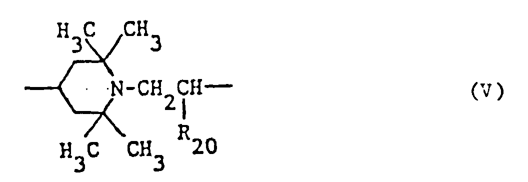 with R₂₀ being hydrogen, C₁-C₈alkyl or phenyl, and q is zero or an integer from 1 to 5, and, if n is 3, A is aliphatic C₄-C₁₈triacyl,aliphatic C₆-C₁₈triacyl substituted by one nitrogen atom, aromatic or heterocyclic triacyl having up to 18 carbon atoms or 1,3,5-triazine-2,4,6-triyl, and, if n is 4, A is aliphatic C₆-C₁₈tetraacyl, aliphatic C₁₀-C₁₈tetraacyl substituted by two nitrogen atoms, aromatic C₁₀-C₁₈tetraacyl or cycloaliphatic C₁₀-C₂₂tetraacyl.

2. A compound of the formula (I) according to claim 1, wherein R₁ is hydrogen, C₁-C₄alkyl, OH, C₆-C₁₂alkoxy, C₅-C₈cycloalkoxy, allyl, benzyl, acetyl or 2-hydroxyethyl.

3. A compound of the formula (I) according to claim 1, wherein R₂ is C₂-C₁₀alkylene, n is 1, 2, 3 or 4 and, if n is 1, A is hydrogen, C₄-C₁₈alkyl, allyl, benzyl, 2-hydroxyethyl or 2-hydroxypropyl, or A is one of the groups of the formula (IIa)-(IIb) or (IId)-(IIh) in which X and Y which can be
identical or different are C₁-C₁₂alkyl, phenyl or a group -OR₁₃, -SR₁₃ or 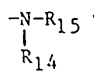 where R₁₃ is C₁-C₁₂alkyl, C₅-C₈cycloalkyl which is unsubstituted or mono-, di- or tri-substituted by C₁-C₄alkyl, C₃-C₁₂alkenyl, phenyl, benzyl or a group of the formula (III), and R₁₄ and R₁₅ which can be identical or different are hydrogen, C₁-C₁₈alkyl, C₅-C₈cycloalkyl which is unsubstituted or mono-, di- or tri-substituted by C₁-C₄alkyl, benzyl, C₂-C₃alkyl substituted in the 2-position or 3-position by OH, by C₁-C₄alkoxy or by di-(C₁-C₄alkyl)-amino, allyl, oleyl, tetrahydrofurfuryl or a group of the formula (III), or the group 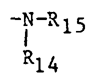 is 1-pyrrolidyl, 1-piperidyl, 4-morpholinyl or 1-hexahydroazepinyl, R₃ is hydrogen, C₁-C₁₇alkyl, C₅-C₈cycloalkyl which is unsubstituted or mono-, di- or tri-substituted by C₁-C₄alkyl, C₂-C₁₇alkenyl, phenyl which is unsubstituted or mono-, di- or tri-substituted by C₁-C₄alkyl and/or an OH group, C₇-C₈-phenylalkyl which is unsubstituted or mono-, di- or tri-substituted on the phenyl by C₁-C₄alkyl and/or an OH group, p is zero or an integer from 1 to 3, R₆ and R₉ are C₁-C₁₈alkyl, C₅-C₈cycloalkyl which is unsubstituted or mono-, di- or tri-substituted by C₁-C₄alkyl, C₃-C₁₈alkenyl or a group of the formula (III), R₅ is a direct bond or C₁-C₁₀alkylene, R₇ is hydrogen or C₁-C₄alkyl, R₈ is -CN or a group -COOR₉ with R₉ being as defined above, R₁₀ and R₁₁ which can be identical or different are as defined above for R₁₄ and R₁₅, R₁₂ is C₁-C₁₂alkyl, phenyl or tolyl, and, if n is 2, A is C₂-C₁₀alkylene, 2-hydroxytrimethylene, xylylene or one of the groups of the formula (IVa)-(IVe) in which Z is as defined above for X and Y, R₁₇ is as defined above for R₅, R₁₈ and R₁₉ are C₂-C₁₀alkylene, C₄-C₁₀alkylene interrupted by 1, 2 or 3 oxygen atoms, cyclohexylenedimethylene, isopropylidenedicyclohexylene, isopropylidenediphenylene or a group of the formula (V) with R₂₀ being hydrogen or methyl, and q is zero or an integer from 1 to 3, and, if n is 3, A is aliphatic C₄-C₈triacyl, a group N-(CH₂CO-)₃, a 1,3,5-triazine-2,4,6-triyl group, benzene-1,2,4-tricarbonyl, benzene-1,3,5-tricarbonyl, N,N',N''-tris-(carbonylmethylene) isocyanurate or N,N',N"-tris-(carbonylethylene) isocyanurate and, if n is 4, A is aliphatic C₆-C₈tetraacyl.

4. A compound of the formula (I) according to claim 1, wherein R₂ is C₂-C₈alkylene, n is 1, 2 or 3 and, if n is 1, A is hydrogen, C₆-C₁₈-alkyl, benzyl or one of the groups of the formula (IIa)-(IIb) or (IId)-(IIh) in which X
and Y which can be identical or different are C₁-C₄alkyl, phenyl or a group -OR₁₃, -SR₁₃ or 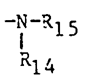 where R₁₃ is C₁-C₈alkyl, cyclohexyl which is unsubstituted or mono-, di- or tri-substituted by C₁-C₄alkyl, allyl, undecenyl, phenyl, benzyl or a group of the formula (III), R₁₄ and R₁₅ which can be identical or different are hydrogen, C₁-C₁₆alkyl, cyclohexyl which is unsubstituted or mono-,di- or tri-substituted by C₁-C₄alkyl, benzyl, C₂-C₃alkyl substituted in the 2-position or 3-position by OH, by C₁-C₄alkoxy, by dimethylamino or by diethylamino, allyl, tetrahydrofurfuryl or a group of the formula (III), or the group 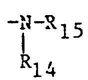 is 4-morpholinyl, R₃ is C₁-C₁₇alkyl, cyclohexyl which is unsubstituted or mono-, di- or tri-substituted by C₁-C₄alkyl, C₂-C₁₀alkenyl, phenyl, t-butylphenyl, 3,5-di-t-butyl-4-hydroxyphenyl, benzyl or 2-(3,5-di-t-butyl-4-hydroxyphenyl)ethyl, p is zero or 1, R₆ and R₉ are C₁-C₁₈alkyl, cyclohexyl which is unsubstituted or mono-, di- or tri-substituted by C₁-C₄alkyl, allyl, undecenyl, oleyl or a group of the formula (III), R₅ is a direct bond or C₁-C₈alkylene, R₇ is hydrogen or methyl, R₈ is -CN or a group -COOR₉ with R₉ being as defined above, R₁₀ and R₁₁ which can be identical or different are as defined above for R₁₄ and R₁₅, and R₁₂ is C₁-C₈alkyl, phenyl or tolyl, and, if n is 2, A is C₂-C₈alkylene, 2-hydroxytrimethylene, xylylene or one of the groups of the formula (IVa)-(IVe) in which Z is as defined above for X and Y, R₁₇ is as defined above for R₅, R₁₈ and R₁₉ are C₂-C₈alkylene, C₄-C₈alkylene interrupted by 1 or 2 oxygen atoms, cyclohexylenedimethylene, isopropylidenedicyclohexylene, isopropylidenediphenylene or a group of the formula (V) with R₂₀ being hydrogen, and q is zero or 1, and, if n is 3, A is aliphatic C₄-C₈-triacyl, benzene-1,2,4-tricarbonyl, benzene-1,3,5-tricarbonyl or a 1,3,5-triazine-2,4,6-triyl group.

5. A compound of the formula (I) according to claim 1, wherein R₂ is C₂-C₆alkylene, n is 1, 2 or 3 and, if n is 1, A ishydrogen or a group of the formula (IIa)- (IIb) or (IId)-(IIe) in which X and Y which can be identical or different are C₁-C₈alkoxy or a group 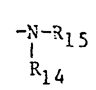 where R₁₄ and R₁₅ which can be identical or different are C₁-C₁₂alkyl, cyclohexyl, benzyl C₂-C₃alkyl substituted in the 2-position or 3-position by methoxy, by ethoxy, by dimethylamino or by diethylamino, allyl, tetrahydrofurfuryl, 2,2,6,6-tetramethyl-4-piperidyl or 1,2,2,6,6-pentamethyl-4-piperidyl, or R₁₄ can also be hydrogen, or the group 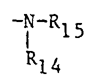 is 4-morpholinyl, R₃ is C₃-C₁₇-alkyl, cyclohexyl, phenyl, 3,5-di-t-butyl-4-hydroxyphenyl or 2-(3,5-di-t-butyl-4-hydroxyphenyl)ethyl, p is zero, R₆ and R₉ are C₂-C₁₈alkyl, cyclohexyl, trimethylcyclohexyl, t-butylcyclohexyl, 2,2,6,6-tetramethyl-4-piperidyl or 1,2,2,6,6-pentamethyl-4-piperidyl, R₅ is a direct bond or C₁-C₄alkylene, R₇ is hydrogen or methyl and R₈ is -CN or a group -COOR₉ with R₉ being as defined above, and, if n is 2, A is one of the groups of the formula (IVa)-(IVd) in which Z is as defined above for X and Y, is is a direct bond or C₁-C₈alkylene and R₁₈ and R₁₉ are C₄-C₈alkylene, cyclohexylenedimethylene, isopropylidenedicyclohexylene or isopropylidenediphenylene, and, if n is 3, A is a 1,3,5-triazine-2,4,6-triyl group.

6. A compound of the formula (I) according to claim 1, wherein R₁ is hydrogen or methyl, R₂ is -(CH₂ 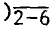 , n is 1, 2 or 3 and if n is 1, A is hydrogen or a group of the formula (IIa)-(IIb) or (IId)-(IIe) in which X and Y which can be identical or different are C₁-C₄alkoxy or a group 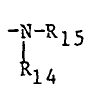 where R₁₄ and R₁₅ which can be identical or different are C₁-C₁₂alkyl, cyclohexyl, 2,2,6,6-tetramethyl-4-piperidyl or 1,2,2,6,6-pentamethyl-4-piperidyl, or R₁₄ can also be hydrogen, R₃ is C₄-C₁₇alkyl, p is zero, R₆ and R₉ are C₂-C₁₈alkyl, cyclohexyl or t-butylcyclohexyl, R₅ is a direct bond, R₇ is hydrogen and R₈ is -CN, and, if n is 2, A is one of the groups of the formula (IVa)-(IVc) in which Z is as defined above for X and Y, R₁₇ is C₂-C₈alkylene and R₁₈ is C₄-C₆alkylene, and, if n is 3, A is a 1,3,5-triazine-2,4,6-triyl group.

7. A compound of the formula (I) according to claim 1, wherein R₁ is hydrogen, R₂ is -(CH₂)₃-, n is 1, 2 or 3 and if n is 1, A is hydrogen or -COC₁₁H₂₃-n, if n is 2, A is -CO-(CH₂)₄-OC- or 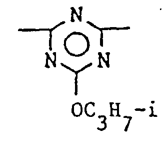 and if n is 3, A is

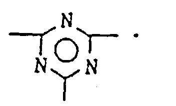

8. A composition containing an organic material susceptible to light-induced, thermal and oxidative degradation and at least one compound of the formula (I) according to claim 1.

9. A composition according to claim 8, wherein the organic material is a synthetic polymer.

10. A composition according to claim 9, which, in addition to the compound of the formula (I), also contains other conventional additives for synthetic polymers.

11. A composition according to claim 8, wherein the organic material is a polyolefin.

12. A composition according to claim 8, wherein the organic material is polyethylene or polypropylene.

13. The use of a compound of the formula (I) according to claim 1 for stabilizing an organic material against light-induced, thermal and oxidative degradation.

## Patentansprüche

1. Verbindung der Formel (I) 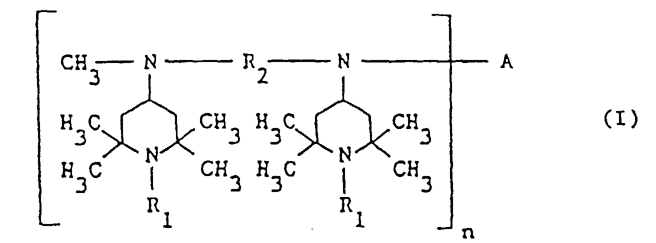 worin R₁ Wasserstoff, C₁-C₈-Alkyl, O^{.}, OH, NO, CH₂CN, C₁-C₁₈-Alkoxy, C₅-C₁₂-Cycloalkoxyl C₃-C₆-Alkenyl, C₇-C₉-Phenylalkyl, unsubstituiert oder am Phenyl mit C₁-C₄-Alkyl mono-, di- oder trisubstituiert, darstellt, oder C₁-C₈-Acyl oder C₂-C₄-Alkyl, substituiert mit OH in der 2-, 3- oder 4-Stellung, darstellt, R₂ C₂-C₁₂-Alkylen darstellt und n 1, 2, 3 oder 4 ist und wenn n 1 ist, A Wasserstoff, C₂-C₁₈-Alkyl, C₃-C₆-Alkenyl, C₇-C₉-Phenylalkyl, unsubstituiert oder am Phenyl mit C₁-C₄-Alkyl mono-, di- oder trisubstituiert, darstellt, oder C₂-C₄-Alkyl, substituiert mit OH in der 2-, 3- oder 4-Stellung, darstellt, oder A eine der Gruppen der Formel (IIa)-(IIb) oder (IId)-(IIh) 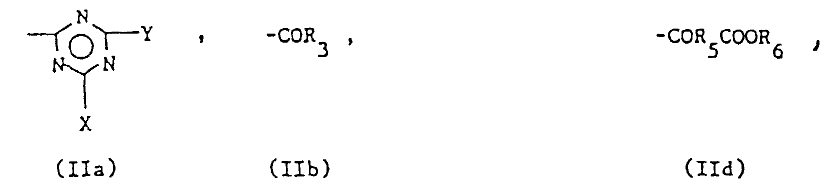 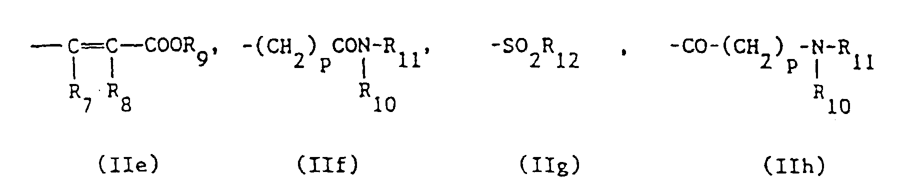 darstellt, worin X und Y, die gleich oder verschieden sein können, C₁-C₁₈-Alkyl, Phenyl, unsubstituiert oder mit C₁-C₄-Alkyl mono-, di- oder trisubstituiert, darstellen oder eine Gruppe -OR₁₃, -SR₁₃ oder 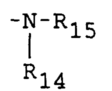 darstellen, worin R₁₃ C₁-C₁₈-Alkyl, C₅-C₁₂-Cycloalkyl, unsubstituiert oder mit C₁-C₄-Alkyl mono-, di- oder trisubstituiert, darstellt oder C₃-C₁₈-Alkenyl, Phenyl, unsubstituiert oder mit C₁-C₄-Alkyl mono-, di- oder trisubstituiert, oder C₇-C₉-Phenylalkyl, unsubstituiert oder am Phenyl mit C₁-C₄-Alkyl mono-, di- oder trisubstituiert, darstellt oder eine Gruppe der Formel (III) 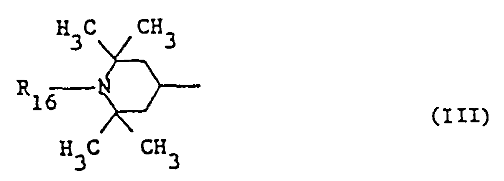 darstellt, worin R₁₆ einer der Definitionen, die für R₁ angegeben sind, entspricht, R₁₄ und R₁₅, die gleich oder verschieden sein können, Wasserstoff, C₁-C₁₈-Alkyl, C₅-C₁₂-Cycloalkyl, unsubstituiert oder mit C₁-C₄-Alkyl mono-, di- oder trisubstituiert, C₇-C₉-Phenylalkyl, unsubstituiert oder mit C₁-C₄-Alkyl mono-, di- oder trisubstituiert, C₂-C₄-Alkyl, substituiert in der 2-, 3- oder 4-Stellung mit OH, mit C₁-C₈-Alkoxy oder mit Di-(C₁-C₄-alkyl)-amino oder C₃-C₁₈-Alkenyl, Tetrahydrofurfuryl oder eine Gruppe der Formel (III) darstellen oder R₁₄ und R₁₅ zusammen mit dem Stickstoffatom, an das sie gebunden sind, einen Teil eines 5- bis 7-gliedrigen heterocyclischen Ringes bilden, R₃ Wasserstoff, C₁-C₁₈-Alkyl, C₅-C₁₂-Cycloalkyl, unsubstituiert oder mit C₁-C₄-Alkyl mono-, di- oder trisubstituiert, C₂-C₁₈-Alkenyl, Phenyl, unsubstituiert oder mit C₁-C₄-Alkyl und/oder einer OH-Gruppe mono-, di- oder trisubstituiert, C₇-C₉-Phenylalkyl, unsubstituiert oder am Phenyl mit C₁-C₄-Alkyl und/oder einer OH-Gruppe mono-, di- oder trisubstituiert, darstellt, p Null oder eine ganze Zahl von 1 bis 5 ist, R₆ und R₉ C₁-C₁₈-Alkyl, C₅-C₁₂-Cycloalkyl, unsubstituiert oder mit C₁-C₄-Alkyl mono-, di- oder trisubstituiert, C₃-C₁₈-Alkenyl oder eine Gruppe der Formel (III) darstellen, R₅ eine direkte Bindung, C₁-C₁₂-Alkylen, Cyclohexylen oder Phenylen darstellt, R₇ Wasserstoff, C₁-C₈-Alkyl oder Phenyl darstellt, R₈ -CN oder eine Gruppe -COOR₉ darstellt, wobei R₉ wie vorstehend definiert ist, R₁₀ und R₁₁, die gleich oder verschieden sein können, wie vorstehend für R₁₄ und R₁₅ definiert sind, und R₁₂ C₁-C₁₈-Alkyl oder Phenyl, unsubstituiert oder mit C₁-C₄-Alkyl mono-, di- oder trisubstituiert, darstellt, und wenn n 2 ist, A C₂-C₁₂-Alkylen, 2-Hydroxytrimethylen, Xylylen oder eine der Gruppen der Formel (IVa)-(IVe) 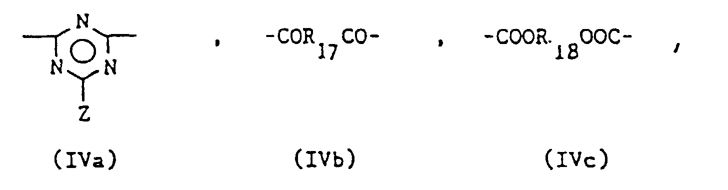 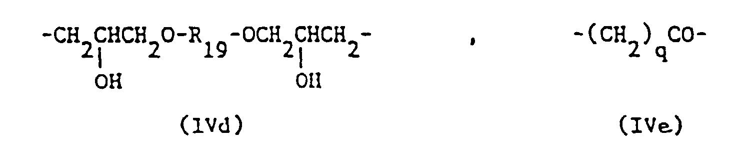 darstellt, worin Z wie vorstehend für X und Y definiert ist, R₁₇ wie vorstehend für R₅ definiert ist, R₁₈ und R₁₉ C₂-C₁₂-Alkylen, C₄-C₁₂-Alkylen, unterbrochen mit 1, 2 oder 3 Sauerstoffatomen, Cyclohexylen, Cyclohexylendimethylen, Methylendicyclohexylen, Isopropylidendicyclohexylen, Phenylen, Isopropylidendiphenylen, Xylylen oder eine Gruppe der Formel (V) 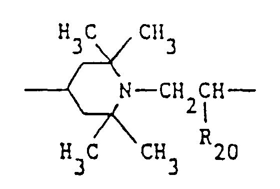 darstellen, wobei R₂₀ Wasserstoff, C₁-C₈-Alkyl oder Phenyl darstellt und q Null oder eine ganze Zahl von 1 bis 5 ist, und wenn n 3 ist, A aliphatisches C₄-C₁₈-Triacyl, aliphatisches C₆-C₁₈-Triacyl, substituiert mit einem Stickstoffatom, aromatisches oder heterocyclisches Triacyl mit bis zu 18 Kohlenstoffatomen oder 1,3,5-Triazin-2,4,6-triyl darstellt, und wenn n 4 ist, A aliphatisches C₆-C₁₈-Tetraacyl, aliphatisches C₁₀-C₁₈-Tetraacyl, substituiert mit zwei Stickstoffatomen, aromatisches C₁₀-C₁₈-Tetraacyl oder cycloaliphatisches C₁₀-C₂₂-Tetraacyl darstellt.

2. Verbindung der Formel (I) nach Anspruch 1, worin R₁ Wasserstoff, C₁-C₄-Alkyl, OH, C₆-C₁₂-Alkoxy, C₅-C₈-Cycloalkoxy, Allyl, Benzyl, Acetyl oder 2-Hydroxyethyl darstellt.

3. Verbindung der Formel (I) nach Anspruch 1, worin R₂ C₂-C₁₀-Alkylen darstellt, n 1, 2, 3 oder 4 ist, und wenn n 1 ist, A Wasserstoff, C₄-C₁₈-Alkyl, Allyl, Benzyl, 2-Hydroxyethyl oder 2-Hydroxypropyl darstellt oder A eine der Gruppen der Formel (IIa)-(IIb) oder (IId)-(IIh) darstellt, worin X und Y, die gleich oder verschieden sein können, C₁-C₁₂-Alkyl, Phenyl oder eine Gruppe -OR₁₃, -SR₁₃ oder 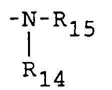 darstellen, worin R₁₃ C₁-C₁₂-Alkyl, C₅-C₈-Cycloalkyl, unsubstituiert oder mit C₁-C₄-Alkyl mono-, di- oder trisubstituiert, C₃-C₁₂-Alkenyl, Phenyl, Benzyl oder eine Gruppe der Formel (III) ist und R₁₄ und R₁₅, die gleich oder verschieden sein können, Wasserstoff, C₁-C₁₈-Alkyl, C₅-C₈-Cycloalkyl, unsubstituiert oder mit C₁-C₄-Alkyl mono-, di- oder trisubstituiert, Benzyl, C₂-C₃-Alkyl, substituiert in der 2-Stellung oder 3-Stellung mit OH, mit C₁-C₄-Alkoxy oder mit Di-(C₁-C₄-alkyl)amino, Allyl, Oleyl, Tetrahydrofurfuryl oder eine Gruppe der Formel (III) darstellen, oder die Gruppe 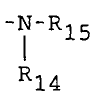 1-Pyrrolidyl, 1-Piperidyl, 4-Morpholinyl oder 1-Hexahydroazepinyl darstellt, R₃ Wasserstoff, C₁-C₁₇-Alkyl, C₅-C₈-Cycloalkyl, unsubstituiert oder mit C₁-C₄-Alkyl mono-, di- oder trisubstituiert, C₂-C₁₇-Alkenyl, Phenyl, unsubstituiert oder mit C₁-C₄-Alkyl und/oder einer OH-Gruppe mono-, di- oder trisubstituiert, C₇-C₈-Phenylalkyl, unsubstituiert oder am Phenyl mit C₁-C₄-Alkyl und/oder einer OH-Gruppe mono-, di- oder trisubstituiert, darstellt, p Null oder eine ganze Zahl von 1 bis 3 ist, R₆ und R₉ C₁-C₁₈-Alkyl, C₅-C₈-Cycloalkyl, unsubstituiert oder mit C₁-C₄-Alkyl mono-, di- oder trisubstituiert, C₃-C₁₈-Alkenyl oder eine Gruppe der Formel (III) darstellen, R₅ eine direkte Bindung oder C₁-C₁₀-Alkylen darstellt, R₇ Wasserstoff oder C₁-C₄-Alkyl darstellt, R₈ -CN oder eine Gruppe -COOR₉ darstellt, wobei R₉ wie vorstehend definiert ist, R₁₀ und R₁₁, die gleich oder verschieden sein können, wie vorstehend für R₁₄ und R₁₅ definiert sind, R₁₂ C₁-C₁₂-Alkyl, Phenyl oder Tolyl darstellt, und wenn n 2 ist, A C₂-C₁₀-Alkylen, 2-Hydroxytrimethylen, Xylylen oder eine der Gruppen der Formel (IVa)-(IVe) darstellt, worin Z wie vorstehend für X und Y definiert ist, R₁₇ wie vorstehend für R₅ definiert ist, R₁₈ und R₁₉ C₂-C₁₀-Alkylen, C₄-C₁₀-Alkylen, unterbrochen mit 1, 2 oder 3 Sauerstoffatomen, Cyclohexylendimethylen, Isopropylidendicyclohexylen, Isopropylidendiphenylen, oder eine Gruppe der Formel (V) darstellen, wobei R₂₀ Wasserstoff oder Methyl darstellt, und q Null oder eine ganze Zahl von 1 bis 3 ist, und wenn n 3 ist, A aliphatisches C₄-C₈-Triacyl, eine Gruppe N-(CH₂CO-)₃, eine 1,3,5-Triazin-2,4,6-triyl-Gruppe, Benzol-1,2,4-tricarbonyl, Benzol-1,3,5-tricarbonyl, N,N',N"-Tris-(carbonylmethylen)isocyanurat oder N,N',N"-Tris-(carbonylethylen)isocyanurat darstellt, und wenn n 4 ist, A aliphatisches C₆-C₈-Tetraacyl darstellt.

4. Verbindung der Formel (I) nach Anspruch 1, worin R₂ C₂-C₈-Alkylen darstellt, n 1, 2 oder 3 ist und, wenn n 1 ist, A Wasserstoff, C₆-C₁₈-Alkyl, Benzyl oder eine der Gruppen der Formel (IIa)-(IIb) oder (IId)-(IIh) darstellt, worin X und Y, die gleich oder verschieden sein können, C₁-C₄-Alkyl, Phenyl oder eine Gruppe -OR₁₃, -SR₁₃ oder 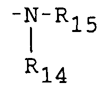 darstellen, worin R₁₃ C₁-C₈-Alkyl, Cyclohexyl, unsubstituiert oder mit C₁-C₄-Alkyl mono-, di- oder trisubstituiert, Allyl, Undecenyl, Phenyl, Benzyl oder eine Gruppe der Formel (III) darstellt, R₁₄ und R₁₅, die gleich oder verschieden sein können, Wasserstoff, C₁-C₁₆-Alkyl, Cyclohexyl, unsubstituiert oder mit C₁-C₄-Alkyl mono-, di- oder trisubstituiert, Benzyl, C₂-C₃-Alkyl, substituiert in der 2-Stellung oder 3-Stellung mit OH, mit C₁-C₄-Alkoxy, mit Dimethylamino oder mit Diethylamino, Allyl, Tetrahydrofurfuryl oder eine Gruppe der Formel (III) darstellen, oder die Gruppe 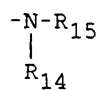 4-Morpholinyl darstellt, R₃ C₁-C₁₇-Alkyl, Cyclohexyl, unsubstituiert oder mit C₁-C₄-Alkyl mono-, di- oder trisubstituiert, C₂-C₁₀-Alkenyl, Phenyl, t-Butylphenyl, 3,5-Di-t-butyl-4-hydroxyphenyl, Benzyl oder 2-(3,5-Di-t-butyl-4-hydroxyphenyl)ethyl darstellt, p Null oder 1 ist, R₆ und R₉ C₁-C₁₈-Alkyl, Cyclohexyl, unsubstituiert oder mit C₁-C₄-Alkyl mono-, di- oder trisubstituiert, Allyl, Undecenyl, Oleyl oder eine Gruppe der Formel (III) darstellt, R₅ eine direkte Bindung oder C₁-C₈-Alkylen darstellt, R₇ Wasserstoff oder Methyl darstellt, R₈ -CN oder eine Gruppe -COOR₉ darstellt, wobei R₉ wie vorstehend definiert ist, R₁₀ und R₁₁, die gleich oder verschieden sein können, wie vorstehend für R₁₄ und R₁₅ definiert sind, und R₁₂ C₁-C₈-Alkyl, Phenyl oder Tolyl darstellt, und wenn n 2 ist, A C₂-C₈-Alkylen, 2-Hydroxytrimethylen, Xylylen oder eine der Gruppen der Formel (IVa)-(IVe) darstellt, worin Z wie vorstehend für X und Y definiert ist, R₁₇ wie vorstehend für R₅ definiert ist, R₁₈ und R₁₉ C₂-C₈-Alkylen, C₄-C₈-Alkylen, unterbrochen mit 1 oder 2 Sauerstoffatomen, Cyclohexylendimethylen, Isopropylidendicyclohexylen, Isopropylidendiphenylen oder eine Gruppe der Formel (V) darstellen, wobei R₂₀ Wasserstoff darstellt, und q Null oder 1 ist, und wenn n 3 ist, A aliphatisches C₄-C₈-Triacyl, Benzol-1,2,4-tricarbonyl, Benzol-1,3,5-tricarbonyl oder eine 1,3,5-Triazin-2,4,6-triyl-Gruppe darstellt.

5. Verbindung der Formel (I) nach Anspruch 1, worin R₂ C₂-C₆-Alkylen darstellt, n 1, 2 oder 3 ist und, wenn n 1 ist, A Wasserstoff oder eine Gruppe der Formel (IIa)-(IIb) oder (IId)-(IIe) darstellt, worin X und Y, die gleich oder verschieden sein können, C₁-C₈-Alkoxy oder eine Gruppe 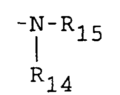 darstellen, worin R₁₄ und R₁₅, die gleich oder verschieden sein können, C₁-C₁₂-Alkyl, Cyclohexyl, Benzyl, C₂-C₃-Alkyl, substituiert in der 2-Stellung oder 3-Stellung mit Methoxy, mit Ethoxy, mit Dimethylamino oder mit Diethylamino, Allyl, Tetrahydrofurfuryl, 2,2,6,6-Tetramethyl-4-piperidyl oder 1,2,2,6,6-Pentamethyl-4-piperidyl darstellen oder R₁₄ ebenfalls Wasserstoff sein kann oder die Gruppe 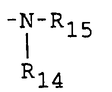 4-Morpholinyl darstellt, R₃ C₃-C₁₇-Alkyl, Cyclohexyl, Phenyl, 3,5-Di-t-butyl-4-hydroxyphenyl oder 2-(3,5-Di-t-butyl-4-hydroxyphenyl)ethyl darstellt, p Null ist, R₆ und R₉ C₂-C₁₈-Alkyl, Cyclohexyl, Trimethylcyclohexyl, t-Butylcyclohexyl, 2,2,6,6-Tetramethyl-4-piperidyl oder 1,2,2,6,6-Pentamethyl-4-piperidyl darstellen, R₅ eine direkte Bindung oder C₁-C₄-Alkylen darstellt, R₇ Wasserstoff oder Methyl darstellt und R₈ -CN oder eine Gruppe -COOR₉ darstellt, wobei R₉ wie vorstehend definiert ist und wenn n 2 ist, A eine der Gruppen der Formel (IVa)-(IVd) darstellt, worin Z wie vorstehend für X und Y definiert ist, R₁₇ eine direkte Bindung oder C₁-C₈-Alkylen darstellt und R₁₈ und R₁₉ C₄-C₈-Alkylen, Cyclohexylendimethylen, Isopropylidendicyclohexylen oder Isopropylidendiphenylen darstellen, und wenn n 3 ist, A eine 1,3,5-Triazin-2,4,6-triylgruppe darstellt.

6. Verbindung der Formel (I) nach Anspruch 1, worin R₁ Wasserstoff oder Methyl darstellt, R₂ -(CH₂)₂₋₆- darstellt, n 1, 2 oder 3 ist, und wenn n 1 ist, A Wasserstoff oder eine Gruppe der Formel (IIa)-(IIb) oder (IId)-(IIe) darstellt, worin X und Y, die gleich oder verschieden sein können, C₁-C₄-Alkoxy oder eine Gruppe 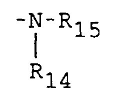 darstellen, worin R₁₄ und R₁₅, die gleich oder verschieden sein können, C₁-C₁₂-Alkyl, Cyclohexyl, 2,2,6,6-Tetramethyl-4-piperidyl oder 1,2,2,6,6-Pentamethyl-4-piperidyl darstellen oder R₁₄ ebenfalls Wasserstoff sein kann, R₃ C₄-C₁₇-Alkyl darstellt, p Null ist, R₆ und R₉ C₂-C₁₈-Alkyl, Cyclohexyl oder t-Butylcyclohexyl darstellen, R₅ eine direkte Bindung darstellt, R₇ Wasserstoff darstellt und R₈ -CN darstellt, und wenn n 2 ist, A eine der Gruppen der Formel (IVa)-(IVc) darstellt, worin Z wie vorstehend für X und Y definiert ist, R₁₇ C₂-C₈-Alkylen darstellt und R₁₈ C₄-C₆-Alkylen darstellt, und wenn n 3 ist, A eine 1,3,5-Triazin-2,4,6-triylgruppe darstellt.

7. Verbindung der Formel (I) nach Anspruch 1, worin R₁ Wasserstoff darstellt, R₂ -(CH₂)₃- darstellt, n 1, 2 oder 3 ist und wenn n 1 ist, A Wasserstoff oder -COC₁₁H₂₃-n darstellt, wenn n 2 ist, A -CO-(CH₂)₄-OC- oder 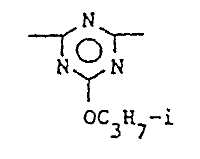 darstellt und wenn n 3 ist, A 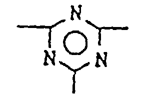 darstellt.

8. Zusammensetzung, enthaltend ein organisches für lichtinduzierten, thermischen und oxidativen Abbau anfälliges Material und mindestens eine Verbindung der Formel (I) nach Anspruch 1.

9. Zusammensetzung nach Anspruch 8, worin das organische Material ein synthetisches Polymer darstellt.

10. Zusammensetzung nach Anspruch 9, die, zusätzlich zu der Verbindung der Formel (I), ebenfalls andere übliche Additive für synthetische Polymere enthält.

11. Zusammensetzung nach Anspruch 8, worin das organische Material ein Polyolefin darstellt.

12. Zusammensetzung nach Anspruch 8, worin das organische Material Polyethylen oder Polypropylen darstellt.

13. Verwendung einer Verbindung der Formel (I) nach Anspruch 1 zum Stabilisieren eines organischen Materials gegen lichtinduzierten, thermischen und oxidativen Abbau.

## Revendications

1. Composés de formule (I) : 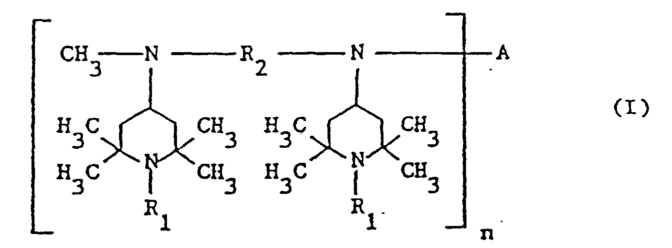 dans laquelle R₁ représente l'hydrogène, alkyle en C₁-C₈, O^{.}, OH, NO, CH₂CN, alcoxy en C₁-C₁₈, cycloalcoxy en C₅-C₁₂, alcényle en C₃-C₆, phénylalkyle en C₇-C₉, qui est non substitué ou mono-, di- ou trisubstitué sur le phényle par alkyle en C₁-C₄, ou représente acyle en C₁-C₈ ou alkyle en C₂-C₄ substitué par OH en positions 2, 3 ou 4, R₂ représente alkylène en C₁-C₁₂ et n vaut 1, 2, 3 ou 4, et si n vaut 1, A représente l'hydrogène, alkyle en C₂-C₁₈, alcényle en C₃-C₆, phénylalkyle en C₇-C₉, qui est non substitué ou mono-, di- ou trisubstitué sur le phényle par alkyle en C₁-C₄, ou représente alkyle en C₂-C₄ substitué par OH en les positions 2, 3 ou 4, ou A est l'un des groupes de formules (IIa) à (IIb) ou (IId) à (IIh) 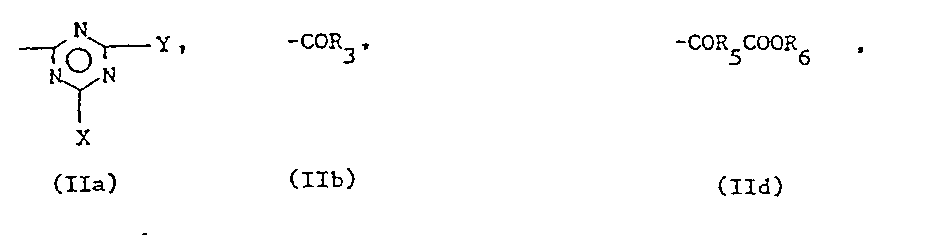 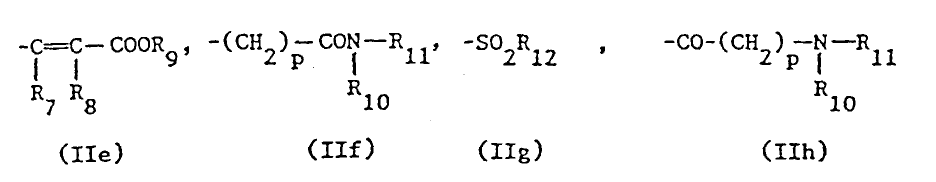 dans lesquelles X et Y, qui peuvent être identiques ou différents, sont alkyle en C₁-C₁₈, phényle qui est non substitué ou mono-, di- ou trisubstitué par alkyle en C₁-C₄, ou un groupe -OR₁₃, -SR₁₃ ou 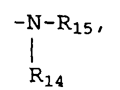 où R₁₃ représente alkyle en C₁-C₁₈, cycloalkyle en C₅-C₁₂ qui est non substitué ou mono-, di- ou trisubstitué par alkyle en C₁-C₄, ou est alcényle en C₃-C₁₈, phényle qui est non substitué ou mono-, di- ou trisubstitué par alkyle en C₁-C₄, ou phénylalkyle en C₇-C₉ qui est non substitué ou mono-, di- ou trisubstitué sur le phényle par alkyle en C₁-C₄, ou est un groupe de formule (III) 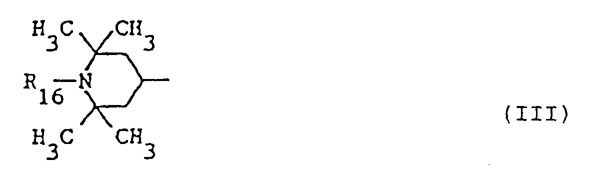 dans laquelle R₁₆ correspond à n'importe laquelle des définitions données pour R₁, R₁₄ et R₁₅, qui peuvent être identiques ou différents, sont l'hydrogène, alkyle en C₁-C₁₈, cycloalkyle en C₅-C₁₂, qui est non substitué ou mono-, di- ou trisubstitué par alkyle en C₁-C₄, phénylalkyle en C₇-C₉, qui est non substitué ou mono-, di- ou trisubstitué par alkyle en C₁-C₄, alkyle en C₂-C₄ substitué en position 2, 3 ou 4 par OH, par alcoxy en C1-C8 ou par di(alkyle en C₁-C₄)amino ou représentent alcényle en C₃-C₁₈, tétrahydrofurfuryle ou un groupe de formule (III), ou R₁₄ et R₁₅, ensemble avec l'atome d'azote auquel ils sont liés, forment un cycle hétérocyclique à 5 à 7 chaînons, R₃ est l'hydrogène, alkyle en C₁-C₁₈, cycloalkyle en C₅-C₁₂, qui est non substitué ou mono-, di- ou trisubstitué par alkyle en C₁-C₄, alcényle en C2-C18, phényle qui est non substitué ou mono-, di- ou trisubstitué par alkyle en C₁-C₄ et/ou un groupe OH, phénylalkyle en C₇-C₉ qui est non substitué ou mono-, di- ou trisubstitué sur le phényle par alkyle en C₁-C₄ et/ou un groupe OH, p vaut 0 ou un nombre entier de 1 à 5, R₆ et R₉ sont alkyle en C₁-C₁₈, cycloalkyle en C₅-C₁₂, qui est non substitué ou mono-, di- ou trisubstitué par alkyle en C₁-C₄, alcényle en C₃-C₁₈ ou un groupe de formule (III), R₅ est une liaison directe, alkylène en C₁-C₁₂, cyclohexylène ou phénylène, R₇ est l'hydrogène, alkyle en C₁-C₈ ou phényle, R₈ est-CN ou un groupe -COOR₉ avec R₉ défini comme ci-dessus, R₁₀ et R₁₁, qui peuvent être identiques ou différents sont définis ci-dessus pour R₁₄ et R₁₅, et R₁₂ est alkyle en C₁-C₁₈ ou phényle qui est non substitué ou mono-, di- ou trisubstitué par alkyle en C₁-C₄, et si n vaut 2, A est alkylène en C₂-C₁₂, 2-hydroxytriméthylène, xylylène ou l'un des groupes de formules (IVa) à (IVe) : 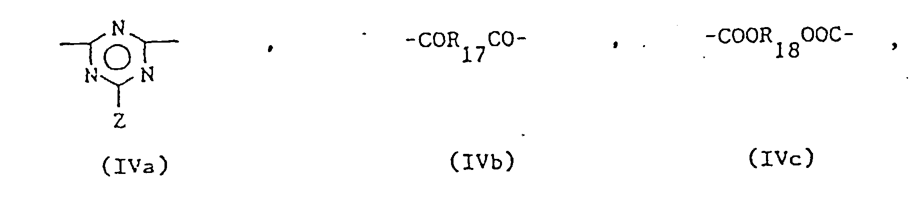 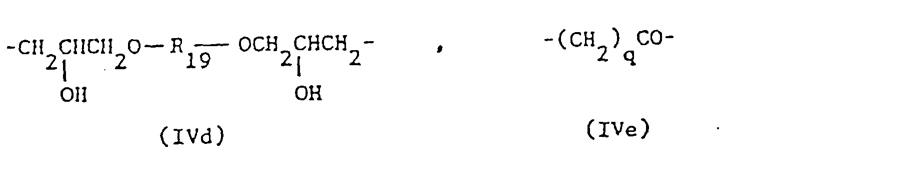 dans lesquelles Z est tel que défini ci-dessus pour X et Y, R₁₇ est tel que défini ci-dessus pour R₅, R₁₈ et R₁₉ sont alkylène en C₂-C₁₂, alkylène en C₄-C₁₂ qui est interrompu par 1, 2 ou 3 atomes d'oxygène, cyclohexylène, cyclohexylènediméthylène, méthylènedicyclohexylène, isopropylidènedicyclohexylène, phénylène, isopropylidènediphénylène, xylylène ou un groupe de formule (V) : 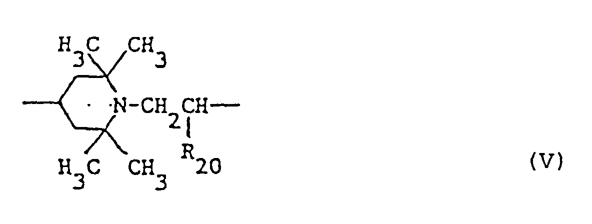 où R₂₀ représente l'hydrogène, alkyle en C₁-C₈ ou phényle, et q est 0 ou un nombre entier de 1 à 5 et, si n vaut 3, A est triacyle en C₄-C₁₈ aliphatique, triacyle en C₆-C₁₈ aliphatique substitué par un atome d'azote, triacyle aromatique ou hétérocyclique ayant 18 atomes de carbone ou 1,3,5,-triazine-2,4,6-triyle, et si n vaut 4, A est tétracyle en C₆-C₁₈ aliphatique, tétracyle en C₆-C₁₈ aliphatique, tétracyle en C₁₀-C₁₈ aliphatique substitué par deux atomes d'azote, tétracyle en C₁₀-C₁₈ aromatique ou tétracyle en C₁₀-C₂₂ cycloaliphatique.

2. Composés de formule (I) selon la revendication 1 où R₁ est l'hydrogène, alkyle en C₁-C₄, OH, alcoxy en C₆-C₁₂, cycloalcoxy en C₅-C₈, allyle, benzyle, acétyle ou 2-hydroxyéthyle.

3. Composés de formule (I) selon la revendication 1, dans lesquels R₂ est alkylène en C₂-C₁₀, n vaut 1, 2, 3 ou 4 et si n vaut 1, A est l'hydrogène, alkyle en C₄-C₁₈, allyle, benzyle, hydroxyéthyle ou 2-hydroxypropyle, ou A est l'un des groupes de formules (IIa) à (IIb) ou (IIb) à (IIh) dans lesquelles X et Y, qui peuvent être identiques ou différents, sont alkyle en C₁-C₁₂, phényle, ou un groupe 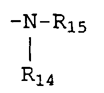 où R₁₃ est alkyle en C₁-C₁₂, cycloalkyle en C₅-C₈, qui est non substitué ou mono-, di- ou trisubstitué par alkyle en C₁-C₄, alcényle en C₃-C₁₂, phényle, benzyle ou un groupe de formule (III), et R₁₄ et R₁₅, qui peuvent être identiques ou différents, sont l'hydrogène, alkyle en C₁-C₁₈, cycloalkyle en C₅-C₈, qui est non substitué ou mono-, di- ou trisubstitué par alkyle en C₁-C₄, benzyle, alkyle en C₂-C₃ substitué en position 2 ou en position 3 par OH, par alcoxy en C₁-C₄ ou par di(alkyl en C₁-C₄)amino, allyle, oléyle, tétrahydrofurfuryle ou un groupe de formule(III), ou le groupe 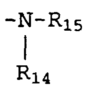 est 1-pyrrolidyle, 4-morpholinyle ou 1-hexahydroazépinyle, R₃ est l'hydrogène, alkyle en C₁-C₁₇, cycloalkyle en C₅-C₈, qui est non substitué ou mono-, di- ou trisubstitué par alkyle en C₁-C₄, alcényle en C₂-C₁₇, phényle qui est non substitué ou mono-, di- ou trisubstitué par alkyle en C₁-C₄ et/ou un groupe OH, phénylalkyle en C₇-C₈ qui est non substitué ou mono-, di- ou trisubstitué sur le phényle par alkyle en C₁-C₄ et/ou un groupe OH, p vaut 0 ou un nombre entier de 1 à 3, R₆ et R₉ sont alkyle en C₁-C₁₈, cycloalkyle en C₅-C₈, qui est non substitué ou mono-, di- ou trisubstitué par alkyle en C₁-C₄, alcényle en C3-C18 ou un groupe de formule (III), R₅ est une liaison directe ou alkylène en C₁-C₁₀, R₇ est l'hydrogène ou alkyle en C₁-C₄, R₈ est -CN ou un groupe -COOR₉ avec R₉ défini comme ci-dessus, R₁₀ et R₁₁, qui peuvent être identiques ou différents sont définis ci-dessus comme pour R₁₄ et R₁₅, R₁₂ est alkyle en C₁-C₁₂, phényle ou tolyle, et si n vaut 2, A est alkylène en C₂-C₁₀, 2-hydroxytriméthylène, xylylène ou l'un des groupes de formules (IVa) à (IVe) dans lesquelles Z est tel que défini ci-dessus pour X et Y, R₁₇ est tel que défini ci-dessus pour R5, R18 et R19 sont alkylène en C₂-C₁₀, alkylène en C₄-C₁₀ interrompu par 1, 2 ou 3 atomes d'oxygène, cyclohexylènediméthylène, isopropylidènecyclohexylène, isopropylidènediphénylène ou un groupe de formule (V) avec R₂₀ étant l'hydrogène ou méthyle, et q vaut 0 ou est un nombre entier de 1 à 3, et si n vaut 3, A est triacyle en C₄-C₈ aliphatique, un groupe N(CH₂CO-)₃, un groupe 1,3,5-triazine-2,4,6-triyle, benzène-1,2,4-tricarbonyle, benzène-1,3,5-tricarbonyle, N,N',N"-tris-(carbonylméthylène) isocyanurate ou N,N',N"-tris-(carbonyléthylène)isocyanurate et, si n vaut 4, est tétracyle en C₆-C₈ aliphatique.

4. Composés de formule (I) selon la revendication 1 où R₂ est alkylène en C₂-C₈, n vaut 1, 2 ou 3 et, si n vaut 1, A est l'hydrogène, alkyle en C₆-C₁₈, benzyle ou l'un des groupes de formules (IIa) à (IIb) ou (IId) à (IIh) dans lesquelles X et Y, qui peuvent être identiques ou différents, sont alkyle en C₁-C₄, phényle, ou un groupe -OR₁₃, -SR₁₃ ou 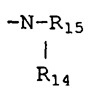 où R₁₃ est alkyle en C₁-C₈, cyclohexyle qui est non substitué ou mono-, di- ou trisubstitué par alkyle en C₁-C₄, allyle, undécényle, phényle, benzyle ou un groupe de formule (III), R₁₄ et R₁₅, qui peuvent être identiques ou différents, sont, l'hydrogène, alkyle en C₁-C₁₆, cyclohexyle qui est non substitué ou mono-, di- ou trisubstitué par alkyle en C₁-C₄, benzyle, alkyle en C₂-C₃ substitué en position 2 ou en position 3 par OH, par alcoxy en C₁-C₄, par diméthylamino ou par diéthylamino, allyle, tétrahydrofurfuryle ou un groupe de formule (III), ou le groupe 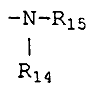 est 4-morpholinyle, R₃ est alkyle en C₁-C₁₇, cyclohexyle qui est non substitué ou mono-, di- ou trisubstitué par alkyle en C₁-C₄, alcényle en C₂-C₁₀, phényle, t-butylphényle, 3,5-di-t-butyl-4-hydroxyphényle, benzyle ou 2-(3,5-di-t-butyl-4-hydroxyphényl)éthyle, p vaut 0 ou 1, R6 et R₉ sont alkyle en C₁-C₁₈, cyclohexyle qui est non substitué ou mono-, di- ou trisubstitué par alkyle en C₁-C₄, allyle, undécényle, oléyle ou un groupe de formule (III), R₅ est une liaison directe ou alkylène en C₁-C₈, R₇ est l'hydrogène ou méthyle, R₈ est -CN ou un groupe -COOR₉ avec R₉ défini comme ci-dessus, R₁₀ et R₁₁, qui peuvent être identiques ou différents, sont définis ci-dessus comme pour R₁₄ et R₁₅, et R₁₂ est alkyle en C₁-C₈, phényle ou tolyle, et si n vaut 2, A est alkylène en C₂-C₈, 2-hydroxytriméthylène, xylylène ou l'un des groupes de formules (IVa) à (IVe) dans lesquelles Z est tel que défini ci-dessus pour X et Y, R₁₇ est tel que défini ci-dessus pour R₅, R₁₈ et R₁₉ sont alkylène en C₂-C₈, alkylène en C₄-C₈ interrompu par 1, 2 ou 3 atomes d'oxygène, cyclohexylènediméthylène, isopropylidènedicyclohexylène, isopropylidènediphénylène ou un groupe de formule (V) avec R₂₀ étant l'hydrogène, et q vaut 0 ou 1, et si n vaut 3, A est triacyle en C₄-C₈ aliphatique, un groupe benzène-1,2,4-tricarbonyle, benzène-1,3,5-tricarbonyle ou 1,3,5-triazine-2,4,6-triyle.

5. Composé de formule (I) selon la revendication 1 où R₂ est alkylène en C₂-C₆, n vaut 1, 2 ou 3, et si n vaut 1, A est l'hydrogène ou l'un des groupes de formules (IIa) à (IIb) ou (IId) à (IIe) dans lesquelles X et Y, qui peuvent être identiques ou différents, sont alcoxy en C1-C8 ou un groupe 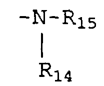 où R₁₄ et R₁₅, qui peuvent être identiques ou différents, sont alkyle en C₁-C₁₂, cyclohexyle, benzyle, alkyle en C₂-C₃ substitué en position 2 ou en position 3 par méthoxy, par éthoxy, par diméthylamino ou par diéthylamino, allyle, tétrahydrofurfuryle, 2,2,6,6-tétraméthyl-4-pipéridyle ou 1,2,2,6,6-pentaméthyl-4-pipéridyle, ou R₁₄ peut aussi être l'hydrogène ou un groupe 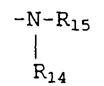 est 4-morpholinyle, R₃ est alkyle en C₃-C₁₇, cyclohexyle, phényle, 3,5-di-t-butyl-4-hydroxyphényle ou 2-(3,5-di-t-butyl-4-hydroxy-phényl)-éthyle, p vaut 0, R₆ et R₉ sont alkyle en C₂-C₁₈, cyclohexyle, triméthylcyclohexyle, t-butylcyclohexyle, 2,2,6,6-tétraméthyl-4-pipéridyle ou 1,2,2,6,6-pentaméthyl-4-pipéridyle, R₅ est une liaison directe ou alkylène en C₁-C₁₄, R₇ est l'hydrogène ou méthyle et R₈ est -CN ou un groupe -COOR₉ avec R₉ défini comme ci-dessus, et si n vaut 2, A est l'un des groupes de formule (IVa) à (IVd) dans lesquelles Z et tel que défini ci-dessus pour X et Y, R₁₇ est une liaison directe ou alkylène en C₁-C₈ et R₁₈ et R₁₉ sont alkylène en C₄-C₈, cyclohexylènediméthylène, isopropylidènecyclohexylène ou isopropylidènediphénylène et, si n vaut 3, A est un groupe 1,3,5-triazine-2,4,6-triyle.

6. Composés de formule (I) selon la revendication 1, où R₁ est l'hydrogène ou méthyle, R₂ est -(CH₂)₂₋₆, n vaut 1, 2 ou 3 et, si n vaut 1, A est l'hydrogène ou l'un des groupes de formules (IIa) à (IIb) ou (IId) à (IIe) dans lesquelles X et Y, qui peuvent être identiques ou différents, sont alcoxy en C₁-C₄ ou un groupe 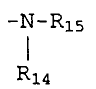 où R₁₄ et R₁₅, qui peuvent être identiques ou différents, sont alkyle en C₁-C₁₂, cyclohexyle, 2,2,6,6-tétraméthyl-4-pipéridyle ou 1,2,2,6,6-pentaméthyl-4-pipéridyle, ou R₁₄ peut aussi être l'hydrogène, R₃ est alkyle en C₄-C₁₇, p vaut 0, R₆ et R₉ sont alkyle en C₂-C₁₈, cyclohexyle ou t-butylcyclohexyle, R₅ est une liaison directe, R₇ est l'hydrogène et R₈ est -CN, et si n vaut 2, A est l'un des groupes de formule (IVa) à (IVc) dans lesquelles Z est tel que défini ci-dessus pour X et Y, R₁₇ est alkylène en C₂-C₈ et R₁₈ est alkylène en C₄-C₆ et, si n vaut 3, A est un groupe 1,3,5-triazine-2,4,6-triyle.

7. Composé de formule (I) selon la revendication 1 où R1 est l'hydrogène, R₂ est -(CH₂)₃-, n vaut 1, 2 ou 3 et si n vaut 1, A est l'hydrogène ou -COC₁₁H₂₃₋ₙ, si n vaut 2, A est -CO-CH₂)₄-OC- ou 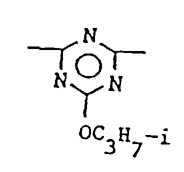 et, si n vaut 3, A est

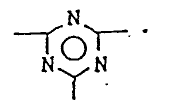

8. Composition contenant une matière organique susceptible de dégradation induite par la lumière, par la chaleur et par oxydation et au moins un composé de formule (I) selon la revendication 1.

9. Composition selon la revendication 8, où la matière organique est un polymère synthétique.

10. Composition selon la revendication 9 qui, en plus des composés de formule (I), contient aussi d'autres additifs classiques pour des polymères synthétiques.

11. Composition selon la revendication 8, où la matière organique est une polyoléfine.

12. Composition selon la revendication 8, où la matière organique est le polyéthylène ou le polypropylène.

13. Utilisation d'un composé de formule (I) selon la revendication 1 pour la stabilisation d'une matière organique contre la dégradation induite par la lumière, par la chaleur et par oxydation.
